# EUROPÄISCHE PATENTSCHRIFT

(11) **EP 1 400 527 B1**
(45) Veröffentlichungstag und Bekanntmachung des Hinweises auf die Patenterteilung: **22.03.2006**
(21) Anmeldenummer: 03018221.6
(22) Anmeldetag: 11.08.2003
(51) Int. Cl.: C07F 9/655

(54) **Chirale Diphosphorverbindungen und deren Übergangsmetallkomplexe**
Chiral diphosphorus compounds and transition metal complexes thereof
Composes diphosphores chiraux et leurs complexes de metaux transitoires

(30) Priorität: 21.08.2002 DE 10238115; 06.09.2002 DE 10241256
(43) Veröffentlichungstag der Anmeldung: 24.03.2004
(73) Patentinhaber: LANXESS Deutschland GmbH, 51369 Leverkusen (DE)
(72) Erfinder: Meseguer, Benjamin, Dr., 43007 Tarragona (ES); Militzer, Hans-Christian, Dr., 51519 Odenthal (DE); Castillon, Sergio, Dr., 43893 Altafulla Tarragona (ES); Claver, Carmen, Prof. Dr., 43893 Altafulla Tarragona (ES); Diaz, Yolanda, Dr., 42006 Tarragona (ES); Aghmiz, Mohamed, Dr., Tetouan, MA 93000 (US); Guiu, Ester, 43001 Tarragona (ES); Aghmiz, Ali, 93000 Tetouan (MA); Masdeu, Anna, 43144 Vallmoll Tarragona (ES)

(56) Entgegenhaltungen:
- EP-A- 0 885 897
- WO-A-95/18787
- DE-A- 10 133 782
- RAJANBABU, T. V. ET AL: "Carbohydrate Phosphinites as Practical Ligands in Asymmetric Catalysis: Electronic Effects and Dependence of Backbone Chirality in Rh-Catalyzed Asymmetric Hydrogenations. Synthesis of R- or S-Amino Acids Using Natural Sugars as Ligand Precursors" JOURNAL OF ORGANIC CHEMISTRY (1997), 62(17), 6012-6028 , XP002216106
- RAJANBABU, T. V. ET AL: "Role of Electronic Asymmetry in the Design of New Ligands: The Asymmetric Hydrocyanation Reaction" JOURNAL OF THE AMERICAN CHEMICAL SOCIETY (1996), 118(26), 6325-6326 , XP002262100

## Beschreibung

Die vorliegende Erfindung betrifft chirale Diphosphorverbindungen und deren Übergangsmetallkomplexe, ein Verfahren zur Herstellung chiraler Diphosphorverbindungen und deren Übergangsmetallkomplexe sowie deren Verwendung in asymmetrischen Synthesen.

Enantiomerenangereicherte chirale Verbindungen sind wertvolle Ausgangssubstanzen zur Herstellung von Agrochemikalien und Pharmazeutika. Dabei hat die asymmetrische Katalyse für die Synthese solcher enantiomerenangereicherten chiralen Verbindungen eine große technische Bedeutung gewonnen.

Die Vielzahl der Publikationen auf dem Gebiet der asymmetrischen Synthese zeigen deutlich, dass Übergangsmetallkomplexe von Diphosphorverbindungen als Katalysatoren in asymmetrisch geführten Reaktionen besonders geeignet sind. Insbesondere haben Übergangsmetallkomplexe von Diphosphorverbindungen als Katalysatoren in asymmetrischen Hydrierungen von C=O, C=N und C=C-Bindungen, Hydrocyanierungen und Hydroformylierungen Eingang in technische Prozesse gefunden.

So ist aus US Patent 5,175,335; Rajan Babu, J. Am. Chem. Soc., 1996, 118, 6325-6326 und Rajan Babu, J. Org. Chem., 1997, 62, 6012-6028 bekannt, enantiomerenangereicherte 1,6-substituierte 3,4-(Bisphosphinito)tetrahydrofurane bzw. deren Übergangsmetallkomplexe für asymmetrische Hydrocyanierungen und Hydrierungen einzusetzen.

Weiterhin ist der Einsatz von enantiomerenangereicherten 3,4-(Bisphosphino)tetrahydrofuranen und deren Übergangsmetallkomplexen in asymmetrischen Hydrierungen aus EP-A 885 897 und A. Terfort, Synthesis, 1992, 10, 951-953 bekannt. Enantiomerenangereicherte 3,4-(Bisphosphito)tetrahydrofurane sind beispielsweise in W. R. Jackson, Aust. J. Chem., 1982, 35, 2069-2075 beschrieben, 3,4-(Phosphino-phosphito)tetrahydrofurane in A. Kless, Tetrahedron: Asymmetry, 1996, 7, 33-36.

Der Nachteil an allen genannten enantiomerenangereicherten 3,4-(Diphosphor)-tetrahydrofuranen ist, dass eine sterische und elektronische Variation des zentralen Tetrahydrofurangerüstes, die für eine gezielte Optimierung und Adaptierung des Liganden und damit des Katalysators für ein vorgegebenes Substrat notwendig ist, nur in sehr begrenztem Umfang und durch zahlreiche, aufwändige Syntheseschritte möglich ist. Diese Nachteile machen eine industrielle Nutzung solcher Liganden und den daraus herstellbaren Katalysatoren unwirtschaftlich.

Es bestand daher das Bedürfnis, ein in seinen sterischen und elektronischen Eigenschaften leicht variierbares Ligandensystem zu entwickeln, dessen Übergangsmetallkomplexe als Katalysatoren in der asymmetrischen Synthese wie insbesondere asymmetrischen Hydrogenierungen neben hoher Enantioselektivität auch hohen Umsatzraten ermöglichen.

Es wurden nun Diphosphorverbindungen der Formel (I) gefunden, 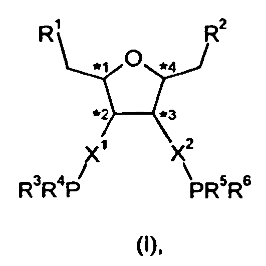 in der
- *1, *2, *3 und *4 jeweils unabhängig voneinander ein stereogenes Kohlenstoffatom markieren, das in R- oder S- Konfiguration vorliegt,
- X¹ und X² jeweils unabhängig voneinander fehlen oder für Sauerstoff stehen und
- R¹ und R² jeweils unabhängig voneinander stehen können für: Wasserstoff, C₁-C₂₀-Alkyl, C₁-C₂₀-Fluoralkyl, C₂-C₂₀-Alkenyl, C₄-C₂₄-Aryl, C₅-C₂₅-Arylalkyl, C₆-C₂₆-Arylalkenyl oder NR⁷R⁸, OR⁸, -(C₁-C₈-Alkyl)-OR⁸, -(C₁-C₈₋Alkyl)-NR⁷R⁸ oder -O₂CR⁸,
   wobei R⁷ und R⁸ jeweils unabhängig voneinander für C₁-C₈-Alkyl, C₅-C₁₅₋Arylalkyl oder C₄-C₁₄-Aryl stehen oder R⁷ und R⁸ zusammen für einen cyclischen Aminorest mit insgesamt 4 bis 20 Kohlenstoffatomen steht,
   oder R¹ und R² jeweils unabhängig voneinander für Reste der Formel (II) stehen

   -R⁹-SiR¹⁰R¹¹R¹² (II)

   in der
   - R⁹: fehlt, für Sauerstoff oder Methylen steht und
   - R¹⁰, R¹¹ und R¹²: jeweils unabhängig voneinander für C₁-C₁₂-Alkyl, C₅-C₁₅₋Arylalkyl oder C₄-C₁₄-Aryl stehen und
- R³, R⁴, R⁵ und R⁶ jeweils unabhängig voneinander für R¹³, OR¹⁴ oder NR¹⁵R¹⁶ stehen, wobei R¹³, R¹⁴, R¹⁵ und R¹⁶ jeweils unabhängig für C₁-C₁₂₋Alkyl, C₅-C₁₅-Arylalkyl oder C₄-C₁₄-Aryl stehen oder NR¹⁵R¹⁶ zusammen für einen cyclischen Aminorest mit 4 bis 20 Kohlenstoffatomen steht oder R³ und R⁴ bzw. R⁵ und R⁶ jeweils zusammen für -O-R¹⁷-O- stehen, wobei R¹⁷ für Reste steht, die ausgewählt sind aus der Gruppe C₂-C₄-Alkylen, 1,2-Phenylen, 1,3-Phenylen, 1,2-Cyclohexylen, 1,1'-Ferrocenylen, 1,2-Ferrocenylen, 2,2'-(1,1'-Binaphtylen), 2,2'-(1,1')-Biphenylen und 1,1'-(Diphenyl-2,2'-methylen)-diyl, wobei die genannten Reste gegebenenfalls einfach oder mehrfach durch Reste substituiert sein können, die ausgewählt sind aus der Gruppe Fluor, Chlor, C₁-C₈-Alkoxy und C₁-C₈-Alkyl.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**Alkyl** beziehungsweise Alkylen beziehungsweise Alkoxy beziehungsweise Alkenyl bedeutet jeweils unabhängig einen geradkettigen, cyclischen, verzweigten oder unverzweigten Alkyl- beziehungsweise Alkylen- beziehungsweise Alkoxybeziehungsweise Alkenyl-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, die isomeren Menthyle, n-Nonyl, n-Decyl und n-Dodecyl, C₁-C₂₀-Alkyl noch weiter darüber hinaus beispielsweise für n-Hexadecyl und n-Octadecyl.

C₁-C₈-Alkoxy steht beispielsweise für Methoxy, Ethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₁₂-Alkoxy weiter darüber hinaus beispielsweise für Adamantoxy, die isomeren Menthoxy-Reste, n-Decoxy und n-Dodecoxy.

C₂-C₂₀-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-butenyl,1-Hexenyl, 1-Heptenyl, 1-Octenyl oder 2-Octenyl.

**Fluoralkyl** bedeutet jeweils unabhängig einen geradkettigen, cyclischen, verzweigten oder unverzweigten Alkyl-Rest, der einfach, mehrfach oder vollständig durch Fluoratome substituiert ist.

Beispielsweise steht C₁-C₂₀-Fluoralkyl für Trifluormethyl, 2,2,2-Trifluorethyl, Pentafluorethyl, Nonafluorbutyl, Perfluoroctyl, Perfluordodecyl und Perfluorhexadecyl.

**Aryl** bedeutet jeweils unabhängig einen heteroaromatischen Rest mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 18 Gerüstkohlenstoffatomen.

Beispiele für carbocyclische aromatische Reste mit 6 bis 18 Gerüstkohlenstoffatomen sind Phenyl, Naphtyl, Phenanthrenyl, Anthracenyl oder Fluorenyl, heteroaromatische Reste mit 5 bis 18 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, C₁-C₁₂-Alkyl, C₁-C₁₂-Fluoralkyl, C₁-C₁₂-Alkoxy, Di(C₁-C₈-alkyl)amino, COO(C₁-C₈-Alkyl), CON(C₁-C₈-Alkyl)₂, COO(C₁-C₈-Arylalkyl), COO(C₄-C₁₄-Aryl), CO(C₁-C₈-Alkyl), C₅-C₁₅-Arylalkyl oder Tri(C₁-C₆-alkyl)siloxyl.

Gleiches gilt analog für **Aryloxy**-Reste.

**Arylalkyl** bedeutet jeweils unabhängig einen geradkettigen, cyclischen, verzweigten oder unverzweigten Alkyl-Rest, der einfach, mehrfach oder vollständig durch Aryl-Reste gemäß obiger Definition substituiert sein kann.

C₅-C₂₅-Arylalkyl steht beispielsweise für Benzyl, Diphenylbenzyl, Triphenylbenzyl (Trityl), 1-Phenylethyl, 1-Phenylpropyl, 2-Phenylpropyl, 1-Phenyl-1-methylethyl, 1-, 2-, 3- oder 4-Phenylbutyl, 1-Phenyl-1-methylpropyl, 1-Phenyl-2-methylpropyl, Phenyl-1,1-dimethylethyl, 1-,2-,3-,4- oder 5-Phenylpentyl, Phenyl-1-methylbutyl, Phenyl-2-methylbutyl, Phenyl-3-methylbutyl, Phenyl-2,2-dimethylpropyl, Phenyl-1-ethylpropyl, 1-Naphthylmethyl, 1-Naphthylethyl, Naphthyl-1-methylethyl, Naphthylbutyl, Naphthyl-1-methylpropyl, Naphthyl-2-methylpropyl, Naphthyl-1,1-dimethylethyl, Naphthylpentyl, Naphthyl-1-methylbutyl, Naphthyl-2-methylbutyl, Naphthyl-3-methylbutyl, Naphthyl-2,2-dimethylpropyl oder Naphthyl-1-ethylpropyl, sowie ihre isomeren oder stereoisomeren Formen.

**Arylalkenyl** bedeutet jeweils unabhängig einen geradkettigen, cyclischen, verzweigten oder unverzweigten Alkenyl-Rest, der einfach, mehrfach oder vollständig durch Aryl-Reste gemäß obiger Definition substituiert sein kann.

C₆-C₂₆-Arylalkenyl steht beispielsweise für 1-Phenylvinyl oder 2-Phenylvinyl.

Im Folgenden werden die bevorzugten Substitutionsmuster für Verbindungen der Formel (I) definiert:
^{*1},^{*2},^{*3},^{*4} definieren zusammen folgende Stereoisomere des zentralen substituierten Furanringes:
(1R,2R,3R,4R), (1R,2R,3R,4S), (1R,2R,3S,4S), (1R,2S,3S,4S), (1R,2S,3R,4S), (1R,2S,3S,4R), (1R,2R,3S,4R), (1S,2S,3R,4S), (1S,2S,3S,4S), (1S,2S,3S,4R), (1S,2S,3R,4R), (1S,2R,3R,4R), (1S,2R,3S,4R), (1S,2R,3R,4S), (1S,2S,3R,4S), (1R,2R,3S,4R), bevorzugt (1R,2R,3R,4R), (1R,2R,3R,4S), (1R,2S,3S,4S), (1R,2S,3S,4R), (1R,2R,3S,4R), (1S,2S,3R,4S), (1S,2S,3S,4S), (1S,2S,3S,4R), (1S,2R,3R,4R), (1S,2R,3R,4S), (1S,2S,3R,4S), (1R,2R,3S,4R).
   - R¹ und R²: stehen bevorzugt jeweils unabhängig voneinander für Wasserstoff, C₁₋C₄-Alkyl, C₄-C₁₄-Aryl, O-R⁸, O₂C-R⁸, wobei R⁸ vorzugsweise für C₁₋C₁₂-Alkyl, C₅-C₂₅-Arylalkyl oder C₄-C₁₄-Aryl steht, oder OSiR¹⁰R¹¹R¹², wobei R¹⁰, R¹¹, und R¹² vorzugsweise jeweils unabhängig für C₁-C₁₂-Alkyl oder C₄-C₁₄-Aryl stehen.
   - R¹ und R²: stehen besonders bevorzugt jeweils unabhängig voneinander für Wasserstoff, tert.-Butoxy, Trityloxy, tert-Butyldimethylsilyloxy, tert-Butyldiphenylsilyloxy, Trimethylsilyloxy, Triethylsilyloxy, Triisopropylsilyloxy, neo-Pentoxy oder 1-Adamantoxy.
Im Rahmen der Erfindung sind solche Verbindungen der Formel (I) jeweils bevorzugt, in denen R¹ und R² identisch sind.
- R³, R⁴, R⁵ und R⁶: stehen bevorzugt jeweils unabhängig voneinander für R¹³, OR¹⁴ oder NR¹⁵R¹⁶, wobei R¹³, R¹⁴, R¹⁵ und R¹⁶ jeweils unabhängig für C₁-C₁₂₋Alkyl oder C₄-C₁₄-Aryl stehen oder NR¹⁵R¹⁶ zusammen für einen cyclischen Aminorest mit 4 bis 12 Kohlenstoffatomen wie zum Beispiel Pyrrolidinyl oder Piperidinyl steht oder R³ und R⁴ bzw. R⁵ und R⁶ jeweils zusammen für -O-R¹⁷-O- stehen, wobei R¹⁷ für Ethylen, 1,2-Phenylen, 1,3-Phenylen, 1,2-Cyclohexylen, 1,1'-Ferrocenylen, zweifach oder vierfach durch C₁-C₈-Alkyl substituiertes 1,1'-(Diphenyl-2,2'-methylen)-diyl, 1,2-Ferrocenylen, 2,2'-(1,1'-Binaphtylen) oder 2,2'-(1,1')-Biphenylen steht, wobei 2,2'-(1,1'-Binaphtylen) oder 2,2'-(1,1')-Biphenylen zumindest in 6,6'-Position durch Reste substituiert ist, die ausgewählt sind aus der Gruppe C₁-C₈-Alkoxy und C₁-C₈₋Alkyl und weiterhin in 5,5'-,4,4'-, 3,3'- oder 2,2'-Position durch Reste substituiert sein kann, die ausgewählt sind aus der Gruppe Fluor, Chlor, C₁₋C₈-Alkoxy und C₁-C₈-Alkyl.
- R³, R⁴, R⁵ und R⁶: stehen besonders bevorzugt jeweils unabhängig voneinander für R¹³, OR¹⁴ oder NR¹⁵R¹⁶, wobei R¹³ und R¹⁴ jeweils unabhängig steht für Methyl, Ethyl, n-Propyl, iso-Propyl, tert.-Butyl, Cyclohexyl, Phenyl, 2-(C₁₋C₈)-alkylphenyl wie o-Tolyl, 3-(C₁-C₈)-alkylphenyl wie m-Tolyl, 4-(C₁-C₈)-alkylphenyl wie p-Tolyl, 2,6-Di-(C₁-C₈)-alkylphenyl wie 2,6-Dimethylphenyl, 2,4-Di-(C₁-C₈)-alkylphenyl wie 2,4-Dimethylphenyl, 3,5-Di-(C₁-C₈)-alkylphenyl wie 3,5-Dimethylphenyl, 3,4,5-Tri-(C₁-C₈)-alkylphenyl wie Mesityl und Isityl, 2-(C₁-C₈)-Alkoxyphenyl wie o-Anisyl und o-Phenetyl, 3-(C₁-C₈)-Alkoxyphenyl wie m-Anisyl und m-Phenetyl, 4-(C₁-C₈)-Alkoxyphenyl wie p-Anisyl und p-Phenetyl, 2,4-Di-(C₁-C₈)-alkoxyphenyl wie 2,4-Dimethoxyphenyl, 2,6-Di-(C₁-C₈)-alkoxyphenyl wie 2,6-Dimethoxyphenyl, 3,5-Di-(C₁-C₈)-Alkoxyphenyl wie 3,5-Dimethoxyphenyl, 3,4,5-Tri-(C₁-C₈)-alkoxyphenyl wie 3,4,5-Trimethoxyphenyl, 3,5-Dialkyl-4-(C₁-C₈)-alkoxyphenyl wie 3,5-Dimethyl-4-anisyl, 3,5-(C₁-C₈)-Dialkyl-4-di-(C₁-C₈)-alkylaminophenyl, 3,5-Dimethyl-4-dimethylamino-phenyl, 4-Di-(C₁₋C₈)-alkylaminophenyl wie 4-Diethylaminophenyl und 4-Dimethylaminophenyl, 3,5-Bis-[(C₁-C₄)-fluoralkyl]phenyl wie 3,5-Bis-trifluormethylphenyl, 2,4-Bis-[(C₁-C₄)-fluoralkyl]phenyl wie 2,4-Bis-trifluormethylphenyl, 4-[(C₁-C₄)-Fluoralkyl]phenyl wie 4-Trifluormethylphenyl und ein-, zwei- drei-, vier- oder fünffach durch Fluor und/oder Chlor substituiertes Phenyl, Fluorenyl oder Naphthyl wie 4-Fluorphenyl und 4-Chlorphenyl und NR¹⁵R¹⁶ als Ganzes für Dimethylamino, Diethylamino, Pyrrolidino oder Diisopropylamino steht. Weiterhin stehen besonders bevorzugt R³ und R⁴ beziehungsweise R⁵ und R⁶ jeweils paarweise für O-R¹⁷-O, wobei R¹⁷ für 1,1'-Bis-(4,6-di-(C₁-C₈-Alkyl)-phenyl)-2,2'-methylen)-diyl wie insbesondere 1,1'-Bis-(4-methyl-6-tert.-butyl-phenyl-2,2'-methylen)-diyl und 1,1'-Bis-(4-methyl-6-(1-methylcyclohexyl)-phenyl-2,2'-methylen)-diyl steht oder wobei R¹⁷ für (R)-1,1'-Biphenyl-2,2'-diyl, (S)-1,1'-Biphenyl-2,2'-diyl, (R)-1,1'-Binaphthyl-2,2'-diyl, (S)-1,1'-Binaphthyl-2,2'-diyl, 1,1'-[Bis-(4-methyl-6-tert.-butyl-phenyl)-2,2'-methylen)]-diyl oder 1,1'-[Bis-(4-methyl-6-(1-methylcyclohexyl)-2,2'-methylen)]-diyl steht.

Im Rahmen der Erfindung sind solche Verbindungen der Formel (I) jeweils bevorzugt, in denen R³ und R⁴ bzw. R⁵ und R⁶ paarweise identisch sind.

Besonders bevorzugte Verbindungen der Formel (I) sind solche der Formeln (Ia) bis (Ii) 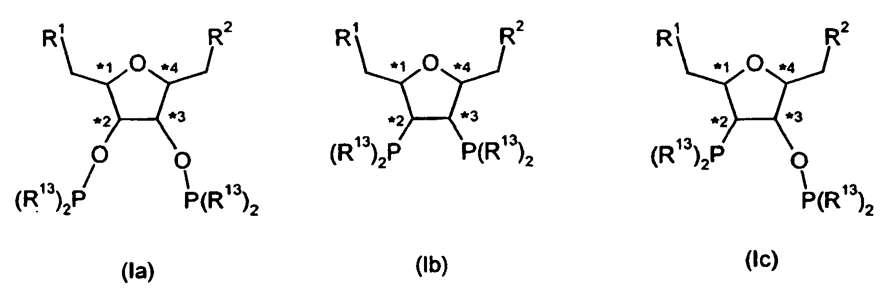 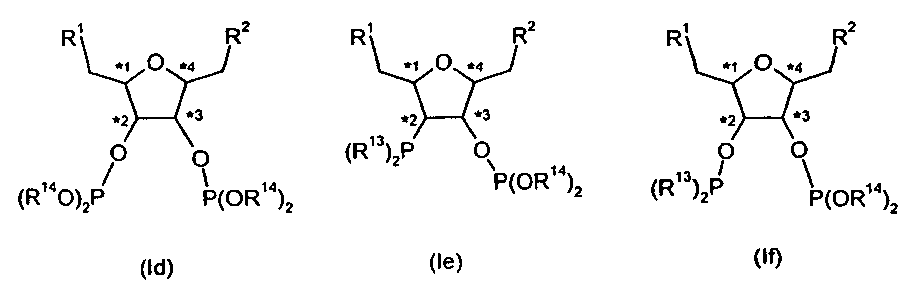 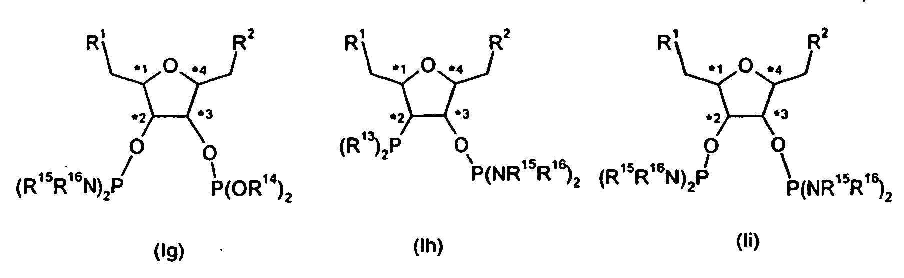 in denen *¹,*²,*³,*⁴, R¹, R², R¹³, R¹⁴, R¹⁵ und R¹⁶ die unter der Formel (I) genannte Bedeutung und Vorzugsbereiche besitzen.

Als Verbindungen der Formel (I) seien genannt:
2,3-bis-*O*-(Diphenylphosphino)-1,6-di-*O*-(triphenylmethyl)-2,5-anhydro-D-mannit, 2,3-bis-*O*-(Diphenylphosphino)-1,6-dideoxy-2,5-anhydro-D-mannit, 2,3-bis-*O*-(Diphenylphosphino)-1,6-di-*O*-(*tert*-butyldiphenylsilyl)-2,5-anhydro-D-mannit, 2,3-bis-*O*-(Diphenylphosphino)-1,6-di-*O*-(triphenylmethyl)-2,5-anhydro-L-iditol, 2,3-bis-*O-*(Diphenylphosphino)-1,6-di-*O*-(*tert*-butyldiphenylsilyl)-2,5-anhydro-L-iditol, 2,3-bis-*O*-(Diphenylphosphino)-1,6-dideoxy-2,5-anhydro-L-iditol, 2,3-bis-*O*-(Di(4-Methoxyphenyl)phosphino)-1,6-di-*O*-(*tert*-butyldiphenylsilyl)-2,5-anhydro-D-mannit, 2,3-bis-*O*-(Di((4-Trifluoromethyl)phenyl)phosphino)-1,6-di-*O*-(*tert*-butyldiphenylsilyl)-2,5-anhydro-D-mannit, 2-*O*-(Di(2,4-Dimethylphenyl)phosphino)-3-*O-*(diphenylphosphino)-1,6-di-*O*-(*tert*-butyldiphenylsilyl)-2,5-anhydro-D-mannit, 2-*O-*(Di(2,4-Dimethylphenyl)phosphino)-3-*O*-(4,8-ditert-butyl-2,10-dimethyl-12H-dibenzo[δ,γ][1,3,2]dioxaphosphocino)-1,6-di-*O*-(*tert*-butyldiphenylsilyl)-2,5-anhydro-D-mannit und 2-*O*-(Di(2,4-Dimethylphenyl)phosphino)-3-*O*-(2,10-dimethyl-4,8-bis(1-methylcyclohexyl)-12H-dibenzo[δ,γ][1,3,2]dioxaphosphocino)-1,6-di-*O*-(*tert-*butyldiphenylsilyl)-2,5-anhydro-D-mannit.

Der Begriff stereoisomerenangereichert umfasst im Sinne der Erfindung insbesondere stereoisomerenreine Verbindungen oder auch Mischungen von stereoisomeren Verbindungen in denen ein Stereoisomeres in einem größeren relativen Anteil als das oder die anderen Stereoisomere, bevorzugt in einem relativen Anteil von 50 % bis 100 Mol-%, besonders bevorzugt 90 bis 100 Mol-% und ganz besonders bevorzugt 98 bis 100 Mol-%, vorliegt.

Die Verbindungen der Formel (I) beziehungsweise (Ia) bis (Ii) können ausgehend von den bekannten 2,5-Anhydrocyclopentosen der Formel (III) hergestellt werden.

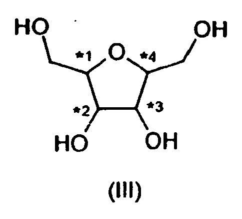

2,5-Anhydrocyclopentosen der Formel (III) sind beispielsweise:
2,5-Anhydro-D-mannit, 2,5-Anhydro-L-mannit, 2,5-Anhydro-L-iditol, 2,5-Anhydro-D-iditol, 2,5-Anhydro-L-glucitol, 2,5-Anhydro-D-glucitol, 2,5-Anhydro-altritol, 2,5-Anhydro-D-altritol, 2,5-Anhydro-galactitol, 2,5-Anhydro-allitol.

Als bevorzugte Verbindungen der allgemeinen Formel (III) seien genant:
2,5-Anhydro-D-mannit und 2,5-Anhydro-L-iditol. Im Rahmen der Erfindung sind insbesondere solche Verbindungen der Formel (I) bevorzugt, die ausgehend von 2,5-Anhydro-D-mannit und 2,5-Anhydro-L-iditol nach den nachfolgend beschriebenen Methoden erhältlich sind.

Die Verbindungen der Formel (III) können durch Umsetzung mit Verbindungen der Formel (IV)

R¹⁸-Hal (IV)

in der R¹⁸ für R⁸, R⁸CO oder OSiR¹⁰R¹¹R¹² stehen und wobei R⁸, R¹⁰, R¹¹ und R¹² die unter der Formel (I) genannte Bedeutung und Vorzugsbereiche besitzen oder R¹⁸ für R¹⁹-SO₂- steht, wobei R¹⁹ für C₁-C₁₂-Alkyl, C₁-C₁₂-Fluoralkyl, C₅-C₂₅-Arylalkyl oder C₄-C₂₄-Aryl steht und
Hal für Chlor, Brom oder Iod steht
in Verbindungen der Formel (V) überführt werden, 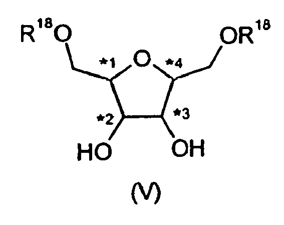 in der R¹⁸ jeweils unabhängig voneinander die unter der Formel (IV) genannte Bedeutung besitzt.

Verbindungen der Formel (V) in denen R¹⁸ für R¹⁹SO₂- steht, können weiterhin durch Umsetzung mit Aminen der Formel (VI)

HNR⁷R⁸ (VI)

in der R⁷ und R⁸ jeweils unabhängig voneinander die unter der Formel (I) angegebenen Bedeutungen und Vorzugsbereiche besitzen in Verbindungen der Formel (VII) überführt werden, 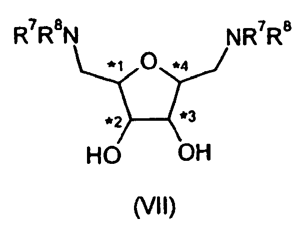 in der R⁷ und R⁸ jeweils unabhängig voneinander die unter der Formel (IV) genannte Bedeutung besitzen.

Weiterhin können Verbindungen der Formel (V) in denen R¹⁸ für R¹⁹SO₂- steht durch Umsetzung mit komplexen Hydriden der Formel (VIII),

Met¹(AlR²⁰ₙR²¹₍₄₋ₙ₎) (VIII)

in der Met¹ für Lithium, Natrium oder Kalium, bevorzugt für Lithium
R²⁰ für Wasserstoff
n für 1, 2, 3 oder 4, bevorzugt für 4 und
R²⁰ für C₁-C₄-Alkyl steht
oder durch Umsetzung mit Organolithiumverbindungen der Formel (IX),

R²⁰-Li (IX)

in der R²⁰ für C₁-C₂₀-Alkyl, C₁-C₂₀-Fluoralkyl, C₂-C₂₀-Alkenyl, C₄-C₂₄-Aryl, C₅-C₂₅₋Arylalkyl, C₆-C₂₆-Arylalkenyl, -(C₁-C₈-Alkyl)-OR⁸, -(C₁-C₈-Alkyl)-NR⁷R⁸ oder (z.B. als cyclisches Acetal) geschütztes -(C₁-C₈-Alkyl)-CO-R⁸ steht zu Verbindungen der Formel (X) umgesetzt werden, 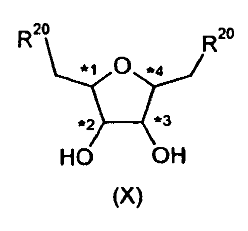 in der R²⁰ die unter den Formeln (VIII) und (IX) angegebene Bedeutung besitzt.

Dabei wird aufgrund der Acidität der freien 2- und 3-Hydroxygruppen vorteilhafterweise ein Überschuss der Organolithiumverbindungen oder der komplexen Hydride eingesetzt oder die 3,4-Dioleinheit in an sich bekannter Weise durch Überführung beispielsweise in ein cyclisches Acetal geschützt und anschließend wieder entschützt.

Die Verbindungen der Formeln (V), (VII) und (X) umfassen zusammen die Verbindungen der Formel (XI), die als Zwischenprodukte zur Herstellung der erfindungsgemäßen Verbindungen der Formel (I) verwendet werden können.

In Formel (XI) 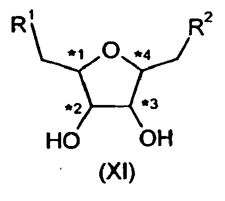 besitzen R¹ und R² die gleiche Bedeutung und Vorzugsbereiche wie unter Formel (I) beschrieben.

Aus den Verbindungen der Formel (XI) können in prinzipiell bekannter Weise (siehe auch Rajan Babu, J. Org. Chem., 1997, 62, 6012-6028) durch Umsetzung mit Verbindungen der Formel (XIIa),

R³R⁴P-Y (XIIa)

in der R³ und R⁴ die gleiche Bedeutung und Vorzugsbereiche besitzen die unter der Formel (I) angegeben sind und
Y für Chlor, Brom, Iod, Dimethylamino oder Diethylamino, vorzugsweise für Chlor steht,
die Verbindungen der Formel (XIII) erhalten werden, 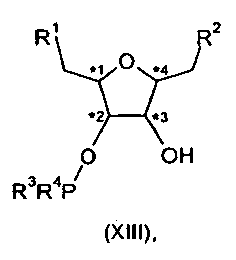 in der R¹, R², R³ und R⁴ die gleiche Bedeutung und Vorzugsbereiche besitzen, wie sie unter der Formel (I) beschrieben sind.

Die Verbindungen der Formel (XIII) können weiterhin mit Verbindungen der Formel (XIIb),

R⁵R⁶P-Y (XIIb)

in der R⁵ und R⁶ die gleichen Bedeutungen und Vorzugsbereiche besitzen, die unter der Formel (I) angegeben sind und Y die gleiche Bedeutung und Vorzugsbereiche besitzt die unter der Formel (XIIa) angegeben sind
zu Verbindungen der Formel (XIV) umgesetzt werden, 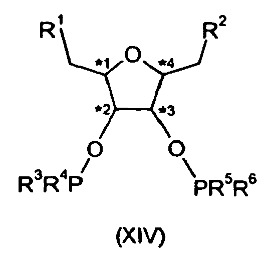 in der R¹, R², R³, R⁴, R⁵ und R⁶ die gleichen Bedeutungen und Vorzugsbereiche besitzen, die unter der Formel (I) angegeben sind.

Sind die Verbindung (XIIa) und (XIIb) identisch, kann die Reaktion auch in einem Schritt durchgeführt werden. Vorteilhafterweise werden die Umsetzungen von (XI) zu (XIV), (XI) zu (XIII) oder (XIII) zu (XIV) in Gegenwart einer Base wie beispielsweise Aminen oder aromatischen Stickstoffbasen wie Triethylamin, Pyridin oder 4-Dimethylaminopyridin durchgeführt. Alternativ dazu kann die Umsetzung auch nach zumindest teilweiser Deprotonierung der Alkoholfunktionen erfolgen.

Als Lösungsmittel für die Umsetzungen eignen sich beispielsweise chlorierte Alkane wie Methylchlorid, alkylische Kohlenwasserstoffe wie z.B Hexan, Cyclohexan, aromatische Kohlenwasserstoffe wie z.B. Toluol, Pyridine, Benzol, Ketone wie z.B. Aceton oder Carbonsäureester wie z.B. Ethylacetat oder Dialkylether, wie z.B. THF oder MTBE. Bevorzugt wird als Lösungsmittel Methylenchlorid eingesetzt.

Auf beschriebene Weise sind insbesondere die Verbindungen der Formeln (Ia), (Id), (If), (Ig) und (Ii) mit der oben genannten Bedeutung und Vorzugsbereichen erhältlich.

Die Verbindungen der Formeln (XIII) und (XIV) sind von der Erfindung ebenfalls umfasst. Dabei gelten für die gleichen Bedeutungen und Vorzugsbereiche die unter der Formel (I) angegeben wurden.

Weiterhin können Verbindungen der Formel (XV) 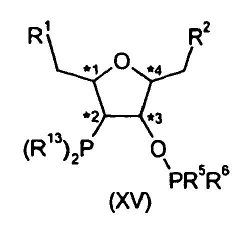 in der R¹, R²,R⁵, R⁶ und R¹³ die unter der Formel (I) angegebenen Bedeutungen und Vorzugsbereiche besitzen durch ein erfindungsgemäßes Verfahren dadurch hergestellt werden, dass Verbindungen der Formel (XVI) 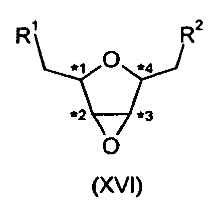 in der R¹ und R² die unter der Formel (I) genannte Bedeutung und Vorzugsbereiche besitzen in Gegenwart von Verbindungen der Formel (XVII),

(R¹³)₂PMet² (XVII)

in der
Met² für Lithium, Natrium oder Kalium steht und
R¹³ die unter der Formel (I) genannte Bedeutung und Vorzugsbereiche besitzt
zu Verbindungen der Formel (XVIII) umsetzt, 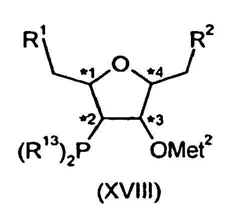 in der R¹, R², Met² und R¹³ die vorstehend genannte Bedeutung besitzen und die Verbindungen der Formel (XVIII) mit Verbindungen der Formel (XIIb) mit der dort angegebenen Bedeutung zu Verbindungen der Formel (XV) umsetzt.

Alternativ können die Verbindungen der Formel (XVII) durch Ansäuern in Verbindungen der Formel (XIX) überführt werden 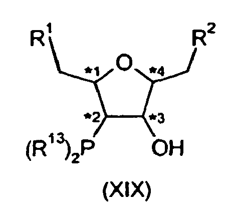 und dann durch Umsetzung mit Verbindungen der Formel (XIIb) in Verbindungen der Formel (XV) überführt werden. Diese Umsetzung kann wie für die Darstellung der Verbindungen der Formel (XIV) beschrieben durchgeführt werden.

Die Verbindungen der Formel (XV) umfasssen insbesondere die erfindungsgemäß bevorzugten Verbindungen der Formeln (Ic), (Ie), (If) und (Ih).

Die Verbindungen der Formel (Ib) 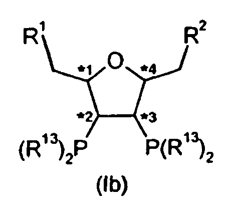 können in an sich bekannter Weise (siehe auch Terfort, Synthesis, 1992, 951-953) beispielsweise dadurch hergestellt werden, dass Verbindungen der Formel (XXa) 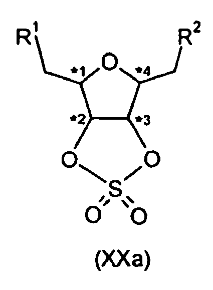 oder Verbindungen der Formel (XXb), 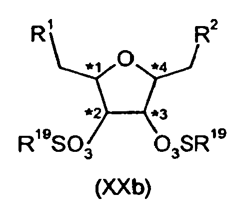 in denen R¹ und R² die unter der Formel (I) genannte Bedeutung und Vorzugsbereiche besitzt und R¹⁹ die unter der Formel (VII) genannte Bedeutung und Vorzugsbereiche besitzt, zunächst mit Phosphiden der Formel (XVII) umsetzt und dabei zu Verbindungen der Formel (XXIa) oder zu Verbindungen der Formel (XXIb) gelangt 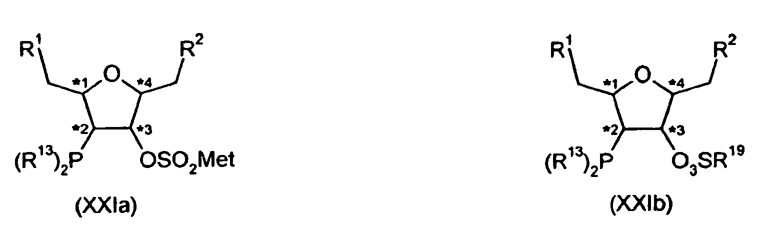 und die Verbindungen der Formel (XXIa) oder Verbindungen der Formel (XXIb) mit Verbindungen der Formel (XVII) zu Verbindungen der Formel (Ib) umsetzt.

Sind in Formel (Ib) die Reste P(R¹³)₂ jeweils identisch, kann die Umsetzung auch in einem Schritt erfolgen.

Die Verbindungen der Formeln (XXIa) und (XXIb) sind von der Erfindung ebenfalls umfasst.

Die Verbindungen der Formel (XXb) sind teilweise literaturbekannt (siehe beispielsweise Tetrahedron: Asymmetry, 2000, 11, 2899 bis 2906). Weitere Verbindungen der Formeln (XXa) und (XXb) sind analog zur Literatur herstellbar. Die Verbindungen der Formel (XXb) sind als besonders geeignete Zwischenverbindungen zur Herstellung von Verbindungen der Formel (I) von der Erfindung ebenfalls umfasst.

Die Erfindung umfasst weiterhin Übergangsmetallkomplexe, die die erfindungsgemäßen Verbindungen der Formel (I) enthalten.

Übergangsmetallkomplexe sind bevorzugt solche von Ruthenium, Osmium, Cobalt, Rhodium, Iridium, Nickel, Palladium, Platin und Kupfer, bevorzugt solche von Ruthenium, Rhodium, Iridium, Nickel, Palladium, Platin und Kupfer.

Die erfindungsgemäßen Übergangsmetallkomplexe eignen sich insbesondere als Katalysatoren. Daher sind von der Erfindung auch Katalysatoren umfasst, die die erfindungsgemäßen Übergangsmetallkomplexe enthalten.

Als Katalysatoren können beispielsweise entweder isolierte Übergangsmetallkomplexe eingesetzt werden oder solche Übergangsmetallkomplexe, die durch Umsetzung von Übergangsmetallverbindungen und Verbindungen der Formel (I) erhältlich sind.

Isolierte Übergangsmetallkomplexe, die die Verbindungen der Formel (I) enthalten, sind vorzugsweise solche, in denen das Verhältnis von Übergangsmetall zu Verbindung der Formel (I) 1:1 betragt.

Bevorzugt sind dabei die erfindungsgemäßen Verbindungen der Formel (XXIII)

[(I)L¹₂M] (XXIII)

in der (I) für Verbindungen der Formel (I) mit der dort genannten Bedeutung und deren Vorzugsbereichen steht und
- M: für Rhodium oder Iridium und
- L¹: jeweils für ein C₂-C₁₂-Alken wie beispielsweise Ethylen oder Cycloocten oder ein Nitril wie beispielsweise Acetonitril, Benzonitril oder Benzylnitril steht, oder
- L¹₂: zusammen für ein (C₄-C₁₂)-Dien wie beispielsweise Bicyclo[2.1.1]hepta-2,5-dien (Norbornadien) oder 1,5-Cyclooctadien steht.

Als Verbindungen der Formel (XXIII) seien genannt:
[Rh(cod)(2,3-bis-*O*-(Diphenylphosphino)-1,6-di-*O*-(triphenylmethyl)-2,5-anhydro-D-mannit)]BF₄, [Rh(cod)(2,3-bis-*O*-(Diphenylphosphino)-1,6-di-*O*-(*tert*-butyldiphenylsilyl)-2,5-anhydro-D-mannit)]BF₄, [Rh(cod)(2,3-bis-*O*-(Diphenylphosphino)-1,6-dideoxy-2,5-anhydro-D-mannit)]BF₄ und [Ir(cod)(2,3-bis-*O*-(Diphenylphosphino)-1,6-di-*O*-(*tert*-butyldiphenylsilyl)-2,5-anhydro-D-mannit)]BF₄.

Bevorzugte Übergangsmetallkomplexe sind solche, die durch Umsetzung von Übergangsmetallverbindungen und Verbindungen der Formel (I) erhältlich sind.

Geeignete Übergangsmetallverbindungen sind beispielsweise solche der Formel (XXIIa)

M(An¹)_{q} (XXIIa)

in der
- M: für Rhodium, Iridium, Ruthenium, Nickel, Paladium, Platin oder Kupfer und
- An¹: für Chlorid, Bromid, Acetat, Nitrat, Methansulfonat, Trifluormethansulfonat oder Acetylacetonat und
- q: für Rhodium, Iridium und Ruthenium für 3, für Nickel, Palladium und Platin für 2 und für Kupfer für 1 steht,
oder Übergangsmetallverbindungen der Formel (XXIIb)

M(An²)_{q}L¹₂ (XXIIb)

in der
- M: für Ruthenium, Iridium, Ruthenium, Nickel, Paladium, Platin oder Kupfer und
- An²: für Chlorid, Bromid, Acetat, Methansulfonat oder Trifluormethansulfonat, Tetrafluoroborat oder Hexafluorophosphat, Perchlorat, Hexafluoroantimonat, Tetra(bis-3,5-trifluromethylphenyl)-borat oder Tetraphenylborat steht und
- q: für Rhodium und Iridium für 1, für Ruthenium, Nickel, Palladium und Platin für 2 und für Kupfer für 1 steht,
- L¹: jeweils für ein C₂-C₁₂-Alken wie beispielsweise Ethylen oder Cycloocten oder ein Nitril wie beispielsweise Acetonitril, Benzonitril oder Benzylnitril steht, oder
- L¹₂: zusammen für ein (C₄-C₁₂)-Dien wie beispielsweise Bicyclo[2.1.1]hepta-2,5-dien (Norbornadien) oder 1,5-Cyclooctadien steht
oder Übergangsmetallverbindungen der Formel (XXIIc)

[ML²An¹₂]₂ (XXIIc)

in der
- M: für Ruthenium und
- L²: für Arylreste wie zum Beispiel Cymol, Mesityl, Phenyl oder Cyclooctadien, Norbomadien oder Methylallyl steht
oder Übergangsmetallverbindungen der Formel (XXIId)

Met³_{q}[M(An²)₄] (XXIId)

in der
- M: für Palladium, Nickel, Iridium oder Rhodium und
- An³: für Chlorid oder Bromid steht und
- Met³: für Lithium, Natrium, Kalium, Ammonium oder organisches Ammonium steht und
- q: für Rhodium und Iridium für 3, für Nickel, Palladium und Platin für 2 steht,
oder Übergangsmetallverbindungen der Formel (XXIIe)

[M(L³)₂]An⁴ (XXIIe)

in der
- M: für Iridium oder Rhodium und
- L³: für (C₄-C₁₂)-Dien wie beispielsweise Bicyclo[2.1.1]hepta-2,5-dien (Norbornadien) oder 1,5-Cyclooctadien steht und
- An⁴: für ein nicht oder schwach koordinierendes Anion wie zum Beispiel Methansulfonat, Trifluormethansulfonat, Tetrafluoroborat, Hexafluorophosphat, Perchlorat, Hexafluoroantimonat, Tetra(bis-3,5-trifluromethylphenyl)-borat oder Tetraphenylborat steht.

Darüber hinaus sind als Übergangsmetallverbindungen beispielsweise Ni(1,5-Cyclooctadien)₂, Pd₂(dibenzylidenaceton)₃, Pd[PPh_{3]4}, Cyclopentadienyl₂Ru, Rh(acac)(CO)₂, Ir(pyridin)2(1,5-Cyclooctadien), Cu(Phenyl)Br, Cu(Phenyl)Cl, Cu(Phenyl)I, Cu(PPh3)₂Br, [Cu(CH₃CN)₄]BF₄ und [Cu(CH₃CN)₄]PF₆ oder mehrkernige verbrückte Komplexe wie beispielsweise [Rh(1,5-cyclooctadien)Cl]₂, [Rh(1,5-cyclooctadien)Br]₂, [Rh(Ethen)₂Cl]₂, [Rh(Cycloocten)₂Cl]₂ geeignet.

Bevorzugt werden als Übergangsmetallverbindungen eingesetzt:
[Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bistrifluormethylphenyl), [Rh(cod)₂]SbF₆, RuCl₂(cod), [(Cymol)RuCl₂]₂, [(Benzol)RuCl₂]₂, [(Mesityl)RuCl₂]₂, [(Cymol)RuBr₂]₂, [(Cymol)RuI₂]₂, [(Cymol)Ru(BF₄)₂]₂, [(Cymol)Ru(PF₆)₂]₂, [(Cymol)Ru(BAr₄)₂]₂ (Ar = 3,5-bistrifluormethylphenyl), [(Cymol)Ru(SbF₆)₂]₂, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bistrifluormethylphenyl), RuCl₃, NiCl₃, RhCl₃, PdCl₂, PdBr₂, Pd(OAc)2, Pd₂(dibenzylidenaceton)₃, Pd(acetylacetonat)₂, CuOTf, CuI, CuCl, Cu(OTf)₂, CuBr, CuI, CuBr₂, CuCl₂, CuI₂, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluormethylphenyl), [Rh(nbd)₂]SbF₆, RuCl₂(nbd), [Ir(nbd)₂]PF₆, [Ir(nbd)₂]ClO₄, [Ir(nbd)₂]SbF₆, [Ir(nbd)₂]BF₄, [Ir(nbd)₂]OTf, [Ir(nbd)₂]BAr₄ (Ar = 3,5-bistrifluormethylphenyl), Ir(pyridin)₂(nbd), [Ru(DMSO)₄Cl₂], [Ru(CH₃CN)₄Cl₂], [Ru(PhCN)₄Cl₂], [Ru(cod)Cl₂]ₙ, [Ru(cod)₄(Methallyl)₂], [Ru(acetylacetonat)₃]

Noch weiter bevorzugt sind [Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bistrifluormethylphenyl), [Rh(cod)₂]SbF₆, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluormethylphenyl), [Rh(nbd)₂]SbF₆, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bistrifluormethylphenyl).

Die Menge der eingesetzten Übergangsmetallverbindungen kann beispielsweise 25 bis 200 mol-% bezogen auf die eingesetzte chirale Diphosphorverbindung der allgemeinen Formel (I) betragen, bevorzugt sind 50 bis 150 mol-%, ganz besonders bevorzugt 75 bis 125 mol-% und noch weiter bevorzugt 100 bis 115 mol-%.

Die Katalysatoren, die die erfindungsgemäßen Übergangsmetallkomplexe enthalten eignen sich insbesondere für den Einsatz in einem Verfahren zur Herstellung von stereoisomerenangereicherten, bevorzugt enantiomerenangereicherten Verbindungen.

Bevorzugt werden die Katalysatoren für asymmetrische 1,4-Additionen, asymmetrische Hydroformylierungen, asymmetrische Hydrocyanierungen, asymmetrische Heck-Reaktionen und asymmetrische Hydrogenierungen eingesetzt, besonders bevorzugt für asymmetrische Hydrogenierungen.

Bevorzugte asymmetrische Hydrogenierungen sind beispielsweise Hydrogenierungen von prochiralen C=C-Bindungen wie zum Beispiel prochirale Enamine, Olefine, Enolether, C=O-Bindungen wie zum Beispiel prochirale Ketone und C=N-Bindungen wie zum Beispiel prochirale Imine. Besonders bevorzugte asymmetrische Hydrogenierungen sind Hydrogenierungen von prochiralen C=C-Bindungen wie zum Beispiel prochirale Enamine, Olefine, und C=N-Bindungen wie zum Beispiel prochirale Imine.

Von der Erfindung ist daher auch ein Verfahren zur Herstellung von stereoisomerenangereicherten, bevorzugt enantiomerenangereicherten Verbindungen durch katalytische Hydrierung von Olefinen, Enaminen, Enamiden, Iminen oder Ketonen umfasst, das dadurch gekennzeichnet ist, dass als Katalysatoren solche verwendet werden, die Übergangsmetallkomplexe von Verbindungen der Formel (I) mit der dort angegebenen Bedeutung verwendet werden.

Die Menge der eingesetzten Übergangsmetallverbindung oder des eingesetzten Übergangsmetallkomplexes kann beispielsweise 0.001 bis 5 mol-% bezogen auf das eingesetzte Substrat betragen, bevorzugt sind 0.001 bis 0,5 mol-%, ganz besonders bevorzugt 0.001 bis 0,1 mol-% und noch weiter bevorzugt 0,001 bis 0,008 mol-%.

In einer bevorzugten Ausführungsform können asymmetrische Hydrogenierungen beispielsweise so durchgeführt werden, dass der Katalysator aus einer Übergangsmetallverbindung und Verbindung der Formel (I) gegebenenfalls in einem geeigneten Lösungsmittel erzeugt wird, das Substrat zugegeben wird und die Reaktionsmischung bei Reaktionstemperatur unter Wasserstoffdruck gesetzt wird.

Besonders bevorzugt kommen für asymmetrische Hydrogenierungen als Metallverbindungen solche der allgemeinen Formel (XXIV) zum Einsatz

[M(L³)₂]An⁴ (XXIV)

wobei M für Rhodium oder Iridium steht und L³ und An die oben genante Bedeutung besitzen,
oder zweikernige Komplexe wie zum Beispiel [Rh(1,5-cyclooctadien)Cl]₂, [Rh(1,5-cyclooctadien)Br]₂, [Rh(Ethen)₂Cl]₂, [Rh(Cycloocten)₂Cl]₂.

Besonders bevorzugte Metallverbindungen für asymmetrische Hydrogenierungen sind [Rh(cod)₂]OTf, [Rh(cod)₂]BF₄, [Rh(cod)₂]PF₆, [Rh(nbd)₂]PF₆, [Rh(nbd)₂]BF₄, und [Rh(Norbomadien)₂]OTf, [Ir(cod)₂]BF₄ und [Ir(cod)₂PF₆].

In einer besonders bevorzugten Ausführungsform werden in einem ausgeheizten Glasautoklaven werden Übergangsmetallverbindung und Verbindung der Formel (I) in entgastem Lösungsmittel gelöst. Man lässt ca. 5 min rühren und gibt anschließend das Substrat in entgastem Lösungsmittel zu. Nach dem Einstellen der jeweiligen Temperatur wird mit H₂-Überdruck hydriert.

Als Lösungsmittel für die asymmetrische Katalyse eignen sich beispielsweise chlorierte Alkane wie Methylchlorid, kurzkettige C₁-C₆-Alkohole wie z.B. Methanol, iso-Propanol oder Ethanol, aromatische Kohlenwasserstoffe wie z.B. Toluol oder Benzol, Ketone wie z.B. Aceton oder Carbonsäureester wie z.B. Ethylacetat.

Die asymmetrische Katalyse wird vorteilhaft bei einer Temperatur von -20°C bis 200°C, bevorzugt 0 bis 100°C und besonders bevorzugt bei 20° bis 70°C durchgeführt.

Der Wasserstoffdruck kann beispielsweise 0,1 bis 200 bar, bevorzugt 0,5 bis 100 und besonders bevorzugt 1 bis 70 bar betragen.

Die erfindungsgemäßen Katalysatoren eignen sich insbesondere in einem Verfahren zur Herstellung von stereoisomerenangereicherten, bevorzugt enantiomerenangereicherten Wirkstoffen in Arzneimitteln und Agrochemikalien, oder Zwischenprodukten dieser beiden Klassen.

Der Vorteil der vorliegenden Erfindung ist, dass die Liganden in effizienter Weise hergestellt werden können und deren elektronische und sterische Eigenschaften ausgehend von einfach verfügbaren Edukten in weitem Masse variabel sind. Weiterhin zeigen die erfindungsgemäßen Liganden und deren Übergangsmetallkomplexe insbesondere in asymmetrischen Hydrogenierungen von C=C-Bindungen und Iminen Tumover-Frequencies (TOFs) von über 1000/h, was weit über vergleichbaren Systemen liegt.

### Beispiele:

### Beispiel 1:

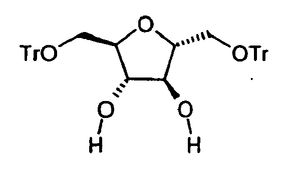

**1,6-Di-*O*-(Triphenylmethyl)-2,5-anhydro-D-mannit (B1):** Eine Mischung von 2.35 g (14.33 mmol) 2,5-Anhydro-D-mannit und 8.79 g (31.53 mmol) Triphenylmethylchlorid in 38 ml Pyridin wurde bei 100°C 12 Stunden gerührt. Nach Abkühlung wurde die Mischung mit CH₂Cl₂ verdünnt, mit HCl aq. (0.78 mol/1) gewaschen, das organische Phase über Na₂SO₄ getrocknet und das Lösungsmittel nachfolgend im Vakuum entfernt. Das Rohprodukt wurde mittels Säulenchromatographie gereinigt (Hexan / Essigester 2:1). Ausbeute: 5.57 g (60% d.Th.).
¹H NMR (400 MHz, CHCl₃) δ, 7.60-7.03 (m, 15H, Ph), 4.12 (m, 1H, H-2), 3.96 (m, 1H, H-3), 3.45 (dd, 1H, J_{6,2}= 3.9 Hz, J_{6,6'}=10.2 Hz, H-6), 3.39 (sa, 1H, OH), 3.22 (dd, 1H, J_{6',2}= 4.2 Hz, J_{6',6}=10.2 Hz, H-6'); ¹³C NMR (100.6 MHz) δ, 143.31.-127.02 (Ph), 87.31 (C(Ph)₃), 83.63 (C-2), 79.45 (C-3), 64.79 (C-6).

### Beispiel 2:

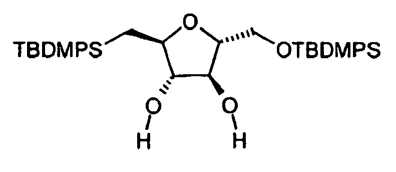

**1,6-Di-*O*-(*tert*-Butyldiphenylsilyl)-2,5-anhydro-D-mannit (B2):** 3 ml (11.66 mmol) *tert*-Butyldiphenylsilylchlorid (TBDMPSCI) wurde bei 0°C zu einer Lösung von 0.87 g (5.3 mmol) 2,5-Anhydro-D-Mannit und 1.5 g (22.28 mmol) Imidazol in 12 ml wasserfreiem DMF getropft. Die Mischung wurde auf Raumtemperatur erwärmt, 25 Stunden weiter gerührt und anschließend das Lösungsmittels im Vakuum entfernt. Die Mischung wurde mit CH₂Cl₂ verdünnt, mit Wasser gewaschen, die organische Phase über Na₂SO₄ getrocknet und das Lösungsmittel nachfolgend im Vakuum entfernt. Das Rohprodukt wurde mittels Säulenchromatographie gereinigt (Hexan / Essigester 4:1). Ausbeute 1.36 g (40% d.Th.).
¹H NMR (400 MHz, CDCl₃) δ, 7.81-7.30 (m, 10H, Ph); 4.25 (m, 1H, H-3); 4.17 (m, 1H, H-2); 4.04 (d, 1H, OH); 3.86 (dd, 1 H, J_{6,2}= 3.7 Hz, J_{6,6'}=11.1 Hz, H-6); 3.75 (dd, 1H, J_{6',2}= 3.2 Hz, J_{6,6'}=11.1 Hz, H-6'); 1.07 (s, 9H, C(CH₃)₃);¹³C NMR (100.6 MHz) δ, 136.10-126.99 (Ph), 87.09 (C-2), 79.71 (C-3), 65.52 (C-6), 26.73 (C(CH₃)₃), 19.02 (C(CH₃)₃).

### Beispiel 3:

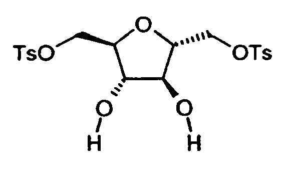

**1,6-Di-*O*-(*p*-Toluolsulfonyl)-2,5-anhydro-D-mannit (B3):** 5.33 g (27.945 mmol) p-Toluolsulfonylchlorid wurden bei 0°C zu einer Lösung von 2.16 g (13.15 mmol) 2,5-Anhydro-D-mannit in 88 ml Pyridin gegeben. Die Mischung wurde auf Raumtemperatur erwärmt und 24 Stunden weiter gerührt. Die Reaktion wurde mit Eiswasser hydrolysiert, mit CH₂Cl₂ extrahiert und die vereinigten organischen Phasen mit 3N HCl und NaCl gewaschen. Nach Trocknen über MgSO₄ wurde das Lösungsmittel nachfolgend im Vakuum entfernt. Das Rohprodukt wurde mittels Säulenchromatographie gereinigt (Hexan / Essigester 1:2). Ausbeute: 3.00 g (48% d.Th.)
¹H NMR (400 MHz, CDCl₃) δ, 7.82-7.31 (m, 4H, Ph); 4.2-3.9 (m, 4H, H-2, H-3, H-6, H-6'); 2.44 (s, 3H, CH3);¹³C NMR (100.6 MHz, CDCl₃) δ, 145.05-126.86 (Ph), 80.42 (C-2), 77.32 (C-3), 68.84 (C-6), 21.81 (CH₃).

### Beispiel 4:

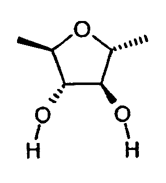

**1,6-Dideoxy-2,5-anhydro-D-mannit (B4):** Zu einer Lösung von 3.60 g (7.63 mmol) **B3** in 20 ml wasserfreiem THF wurden 0.86 g (22.66 mmol) LiA1H₄ zugegeben und bei Ruckfluss 2 Stunde gerührt. Nach Abkühlung wurde Arberlite® IR-120(plus) zugegeben und weiter gerührt bis die Wasserstoffentwicklung beendet war. Die Mischung wurde durch Celite® abfiltriert und das Lösungsmittel nachfolgend im Vakuum entfernt. Das Rohprodukt wurde mittels Säulenchromatographie gereinigt (CH₂Cl₂ / MeOH= 12:1). Ausbeute: 0.84 g (84% d.Th.).
¹H NMR (400 MHz, CDCl₃) δ, 3.84 (m, 1H, H-2), 3.64 (m, 1H, H-3), 1.27 (d, 1H, J_{6,2}= 6 Hz, H-6);¹³C NMR (100.6 MHz; CDCl₃) δ, 84.18 (C-2), 79.29 (C-3), 19.61 (C-6).

### Beispiel 5:

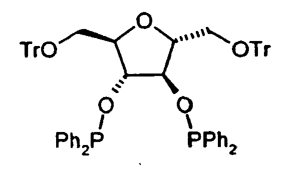

**2,3-bis-*O*-(Diphenylphosphino)-1,6-di-*O*-(triphenylmethyl)-2,5-anhydro-D-mannit (B5).** Zu einer Lösung von 0.63 g (1.0 mmol) **B1** in 5 ml wasserfreiem, entgastem CH₂Cl₂ wurden 0.61 ml (4.4 mmol) wasserfreies Et₃N zugegeben. Bei -15°C wurde eine Lösung von 0.39 ml (2.2 mmol) Diphenylchlorophosphin im 3 ml CH₂Cl₂ langsam zugetropft. Nach 15 Minuten Rühren bei -15°C wurde Ethylether zugegeben, die Salze durch Celite® abfiltriert und das Lösungsmittel nachfolgend im Vakuum entfernt. Das Rohprodukt wurde mittels Säulenchromatographie unter Argon gereinigt (Hexan / Ethylacetat 15:1). Ausbeute 0.51 g (51% d.Th.).
[α]_{D}= + 0.4 (c 1.04, CHCl₃); ¹H NMR (400 MHz, CHCl₃) δ, 760.-7.02 (m, 25H, Ph), 4.62 (m, 1H, H-3), 4.29 (m, 1H, H-2), 3.198 (d, 2H, J_{6, 2}= 5.6 Hz, H-6, H-6'); ¹³C NMR (100.6 MHz) δ, 144.33-127.06 (Ph), 86.901 (C(Ph)₃), 86.14 (m, C-3), 83.51 (m, C-2), 64.25 (s, C-6); ³¹P NMR (161.974 MHz, CDCl₃) δ, 116.23 (s). Anal calcd for C₆₈H₅₈O₅P₂: C, 80.29; H, 5.74; found C, 79.99; H, 5.72.

### Beispiel 6:

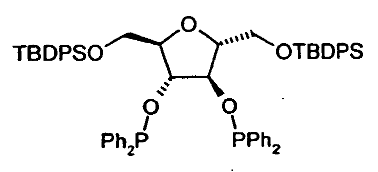

**2,3-bis-*O*-(Diphenylphosphino)-1,6-di-*O*-(*tert*-butyldiphenylsilyl)-2,5-anhydro-D-mannit (B6):** Dieses Produkt wurde analog zu Beispiel 5 bei -25°C aus **B2** hergestellt. Ausbeute: 0.816 g (50%), weißes Öl.
[α]_{D}= + 9.9 (c 1.8, CHCl₃); ¹H NMR (400 MHz, CDCl₃) δ, 7.81-7.02 (m, 20H, Ph), 4.88 (m, 1H, H-3), 4.18 (m, 1H, H-2), 3.76 (dd, 1H, JH-6), 3.63 (d, 1H, H-6'); 1.02 (s, 9H, C(CH₃)₃);¹³C NMR (100.6 MHz, CDCl₃) δ, 144.02-126.10 (Ph), 86.14 (m, C-3), 83.51 (m, C-2), 64.25 (s, C-6), 26.79 (C(CH₃)3), 19.26 (C(CH₃)₃); ³¹P NMR (161.974 MHz, CDCl₃) δ, 116.23 (s). Anal calcd for C₆₂H₆₆O₅P₂Si₂: C, 73.78; H, 6.59; found C, 73.65; H, 6.57.

### Beispiel 7:

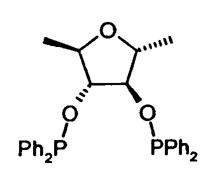

**2,3-bis-*O*-(Diphenylphosphino)-1,6-dideoxy-2,5-anhydro-D-mannit (B7): Dieses** Produkt wurde analog zu Beispiel 5 aus **B4** hergestellt. Ausbeute: 58% d.Th.
[α]_{D}= - 20.4 (*c* 1.04, CHCl₃); ¹H NMR (400 MHz, CDCl₃) δ, 7.60-7.02 (m, 10H, Ph), 4.26 (m, 1H, H-3), 4.12 (m, 1H, H-2), 1.21 (d, 3H, J_{6,2}=6.4 Hz, H-6);¹³C NMR (100.6 MHz, CDCl₃) δ, 142.12-127.08 (Ph), 90.74 (m, C-2), 78.35 (m, C-3), 19.15 (s, C-6); ³¹P NMR (161.974 MHz, CDCl₃) δ, 114.39 (s). Anal calcd for C₃₀H₃₀O₃P₂: C, 71.99; H, 6.04; found C, 72.22; H, 6.06.

### Beispiel 8:

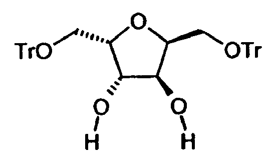

**1,6-di-*O*-(Triphenylmethyl)-2,5-anhydro-L-iditol (B8):** Dieses Produkt wurde analog zu Beispiel 1 ausgehend von 2,5-Anhydro-L-iditol hergestellt.
¹H NMR (400 MHz, CHCl₃) δ, 7.60-7.02 (m, 15H, Ph), 4.42 (m, 1H, H-2), 4.25 (m, 1H, H-3), 3.48 (dd, 1H, J_{6,2}= 5.4 Hz, J_{6,6'}= 9.6 Hz, H-6), 3.40 (dd, 1H, J_{6',2}= 3.8 Hz, J_{6',6}= 9.8 Hz, H-6'); 3.24 (sa, 1H, OH), ¹³C NMR (100.6 MHz) δ, 143.20.-127.01 (Ph), 87.20 (C(Ph)₃), 78.78 (C-2), 78.65 (C-3), 62.78 (C-6).

### Beispiel 9:

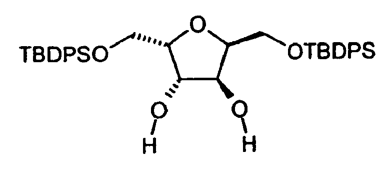

**1,6-di-*O*-(*tert*-Butyldiphenylsilyl)-2,5-anhydro-L-iditol (B9):** Dieses Produkt wurde analog zu Beispiel 2 ausgehend von 2,5-anhydro-L-iditol hergestellt.
¹H NMR (400 MHz, CDCl₃) δ, 7.81-7.32 (m, 10H, Ph); 4.26 (m, 1H, H-2); 4.38 (m, 1H, H-3); 4.07 (d, 1H, J_{6,2}= 4.4 Hz, J_{6,6'}= 10.8 Hz, H-6); 4.05 (d, 1H, J_{6',2}= 3.2 Hz, J_{6',6}= 10.8 Hz, H-6'); 3.99 (d, 1H, OH); 1.06 (s, 9H, C(CH₃)₃);¹³C NMR (100.6 MHz) δ, 135.52-127.6 (Ph), 79.74 (C-2), 78.95 (C-3), 63.74 (C-6), 26.79 (C(CH₃)₃), 19.19 (C(CH₃)₃).

### Beispiel 10:

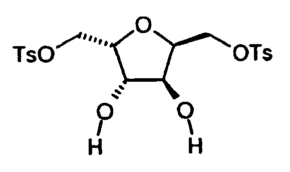

**1,6-di-*O*-(*p*-Toluensulfonyl)-2,5-anhydro-L-iditol (B10):** Dieses Produkt wurde analog zu Beispiel 3 ausgehend von 2,5-anhydro-L-iditol hergestellt.
¹H NMR (400 MHz, CDCl₃) δ, 7.83-7.21 (m, 4H, Ph); 4.42-3.91 (m, 5H, H-2, H-3, H-6, H-6', OH); 2.44 (s, 3H, CH₃);¹³C NMR (100.6 MHz, CDCl₃) δ, 145.11-127.83 (Ph), 77.83 (C-2), 76.54 (C-3), 67.22 (C-6), 21.82 (CH₃).

### Beispiel 11:

### 1,6-Dideoxy-2,5-anhydro-L-iditol (B11):

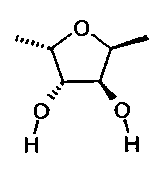

Dieses Produkt wurde analog zu Beispiel 4 ausgehend von **B10** hergestellt.
¹H NMR (400 MHz, CDCl₃) δ, 4.22 (m, 1H, H-2), 3.90 (m, 1H, H-3), 1.18 (d, 1H, J_{6,2}= 6.6 Hz, H-6); ¹³C NMR (100.6 MHz; CDCl₃) δ, 79.66 (C-2), 77.30 (C-3), 14.68 (C-6).

### Beispiel 12:

### 2,3-bis-O-(Diphenylphosphino)-1,6-di-O-(triphenylmethyl)-2,5-anhydro-L-iditol (B12).

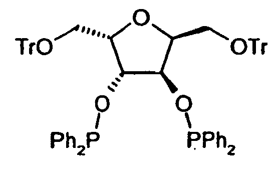

Dieses Produkt wurde analog zu Beispiel 5 ausgehend von **B8** hergestellt.
¹H NMR (400 MHz, CHCl₃) δ, 750.-7.51 (m, 25H, Ph), 4.30 (m, 1H, H-2), 4.37 (m, 1H, H-3), 3.48 (dd, 1H, J_{6,2}= 9.4 Hz, J_{6,6'}= 9.4 Hz, H-6); 3.18 (dd, 1H, J_{6',2}= 5.8 Hz, J_{6',6}= 9.4 Hz, H-6') ¹³C NMR (100.6 MHz) δ, 143.91-126.62 (Ph), 86.77 (C(Ph)₃), 82.64 (m, C-3), 79.654 (d, C-2), 62.82 (s, C-6); ³¹P NMR (161.974 MHz, CDCl₃) δ, 114.12 (s).

### Beispiel 13:

### 2,3-bis-O-(Diphenylphosphino)-1,6-di-O-(tert-butyldiphenylsilyl)-2,5-anhydro-L-iditol (B13):

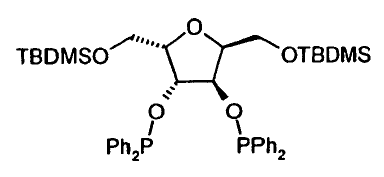

Dieses Produkt wurde analog zu Beispiel 5 ausgehend von **B9** hergestellt.
¹H NMR (400 MHz, CDCl₃) δ, 7.81-7.09 (m, 20H, Ph), 4.51 (m, 1H, H-3), 4.21 (m, 1H, H-2), 3.85 (dd, 1H, J_{6,2}= 7.4 Hz, J_{6,6'}= 10.0 Hz H-6), 3.72 (dd, 1H, J_{6',2}= 5.8 Hz, J_{6',6}= 10.0 Hz, H-6'), 0.94 (s, 9H, C(CH₃)₃);¹³C NMR (100.6 MHz, CDCl₃) δ, 135.51-127.43 (Ph), 83.09 (m, C-3), 80.68 (d, C-2), 61.73 (s, C-6), 26.92 (C(CH₃)₃), 19.25 (C(CH₃)₃); ³¹P NMR (161.974 MHz, CDCl₃) δ, 115.74 (s).

### Beispiel 14:

### 2,3-bis-O-(Diphenylphosphino)-1,6-dideoxy-2,5-anhydro-L-iditol (B14):

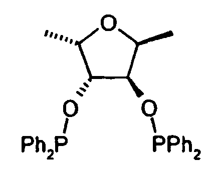

Dieses Produkt wurde analog zu Beispiel 5 ausgehend von **B11** hergestellt.
¹H NMR (400 MHz, CDCl₃) δ, 7.63-7.21 (m, 10H, Ph), 4.32 (m, 1H, H-2), 4.23 (m, 1H, H-3), 1.20 (d, 3H, J_{6,2}=6.4 Hz, H-6);¹³C NMR (100.6 MHz, CDCl₃) δ, 142.05-127.99 (Ph), 84.97 (m, C-3), 76.02 (m, C-2), 15.18 (s, C-6); ³¹P NMR (161.974 MHz, CDCl₃) δ, 114.05 (s).

### Beispiel 15:

**[Rh(cod)(B5)]BF**_{**4**} **(B15):** 0.030 g (0.073 mmol) [Rh(cod)₂]BF₄ wurden in 10 ml CH₂Cl₂ gelöst. Zu dieser Lösung wurde einer Lösung 0.090 g (0.088 mmol) Verbindung **B5** in 3 ml CH₂Cl₂ zugegeben und die erhaltene Lösung 30 min gerührt. Das Lösungsmittel wurde im Vakuum entfernt und das Rohprodukt mit wasserfreiem Hexan und mit Ethylether gewaschen. Ausbeute: 0.042 g (43% d.Th.).
[α]_{D}= + 119.41 (c 1.05, CHCl₃); ¹H NMR (400 MHz, CDCl₃) δ, 7.60-7.02 (m, 25H, Ph), 5.36 (m, 1H, H-3), 4.70 (m, 2H, CH(cod)); 4.47 (m, 1H, H-2), 3.58 (dd, 1H, J_{6,2}= 2.8 Hz, J_{6,6'}= 10.8 Hz, H-6), 3.18 (dd, 1H, J_{6,2}= 3.2 Hz, J_{6,6'}= 10.8 Hz, H-6'); 2.42-2.00 (m, 4H, CH₂(cod)); ¹³C NMR (100.6 MHz) δ, 144.10-126.05 (Ph, cod), 87.00 (C(Ph)₃), 82.67 (s, C-3), 82.45 (m, C-2), 63.33 (s, C-6); ³¹P NMR (161.974 MHz, CDCl₃) δ, 122.61 (d, J_{P,Rh}=166.18 Hz)).

### Beispiel 16:

**[Rh(cod)(B6)]BF**_{**4**} **(B16):** Dieses Komplex wurde analog zu Beispiel 15 ausgehend von B6 hergestellt. Ausbeute: 68% d.Th.
[α]_{D}= (c, CHCl₃); ¹H NMR (400 MHz, CDCl₃) δ, 7.82-7.21 (m, 20H, Ph), 5.24 (m, 1H, H-3), 4.77 (m, 1H, CH(cod)); 4.67 (m, 1H, CH(cod)); 4.19 (m, 1H, H-2), 3.85 (dd, 1H, J_{6, 2}= 2.4 Hz, J_{6, 6'}= 11.6 Hz, H-6), 3.69 (dd, 1H, J_{6', 2}= 3.2 Hz, J_{6, 6'}= 11.6 Hz, H-6'); 2.41-2.20 (m, 4H, CH₂(cod)); 1.04 (s, 9H, C(CH₃)₃); ¹³C NMR (100.6 MHz, CDCl₃) δ, 134.10-126.07 (Ph, cod), 82.53 (m, C-2), 81.23 (s, C-3), 63.13 (s, C-6), 26.95 (C(CH₃)₃), 19.44 (C(CH₃)₃); ³¹P NMR (161.974 MHz, CDCl₃) δ, 125.136 (d, J_{P,Rh}= 167.97 Hz).

### Beispiel 17:

**[Rh(cod)(B7)]BF**_{**4**} **(B17):** Dieses Komplex wurde analog zu Beispiel 15 ausgehend von B7 hergestellt. Ausbeute: 55% d.Th..
¹H NMR (400 MHz, CDCl₃) δ, 7.81-7.42 (m, 10H, Ph), 4.73 (m, 1H, CH(cod)); 4.64 (m, 1H, CH(cod));4.39 (m, 1H, H-3), 3.91 (m, 1H, H-2), ); 2.61-2.20 (m, 4H, CH₂(cod)); 1.13 (d, 3H, J_{6,2}=6.0 Hz, H-6);¹³C NMR (100.6 MHz, CDCl₃) δ, 133.15-128.03 (Ph, cod), 85.76 (s, C-3), 75.68 (m, C-2), 18.64 (s, C-6); ³¹P NMR (161.974 MHz, CDCl₃) δ, 127.39 (d, J_{P,Rh}= 169.42 Hz).

### Beispiel 18:

**[Ir(cod)(B6)]BF**_{**4**} **(B18):** Zu einer auf -80°C gekühlten Lösung des 40 mg (0.08 mmol) [Ir(cod)_{2]}BF₄ in 2 ml CH₂Cl₂, wurde eine Lösung 102 mg (0.1 mmol) der Verbindung B6 in 2 ml CH₂Cl₂ zugetropft. Die erhaltene gelbe Lösung wurde auf 0°C erwärmt und 15 Minuten weiter gerührt. Das Lösungsmittel wurde teilweise im Vakuum entfernt, 30 ml Ethylether wurden zugegeben und der Feststoff abfiltriert und gewaschen. Ausbeute: 98,6 mg, 86% d.Th.
¹H NMR (400MHz, CDCl₃) δ, 7,44-7,24 (m, 50H, CH arom), 5,35 (m, 2H, CH), 5,17 (m, 1H, CH); 4,52 (m, 2H, CH); 4,41 (m, 4H, CH₂); 3,60 (dd, *J*= 2.6 Hz, *J*= 10,4 Hz, 2H, CH₂), 3,21 (dd, *J=* 2.6 Hz, *J=* 10,4 Hz, 2H, CH₂), 2,42 (m, 2H, cod), 2,21 (m, 2H,), 2,06 (m, 2H), 1,67 (m, 2H). ¹³C NMR (100.6 MHz) δ, 143.6 (C arom.), 132,4 (s, arom); 32,0 (s, arom); 131,7 (t, arom.), 131,2 (t, arom.), 129,1 (m, arom.), 128,7 (m, arom.), 128,68 (s, CH arom.), 128,1 (s, CH arom.), 127,4 (s, CH arom), 95,5 (s, CH), 83,3 (s, CH), 87,2 (s, C), 83,1 (s, CH), 82,4 (s, CH), 63.7 (CH₂), 31,3 (s, CH₂), 31,1 (s, CH₂). ³¹P NMR (161.9 MHz, CDCl₃) δ, 104.6.

### Beispiele 19-41:

### Rhodium-katalysierte Hydrogenierung von Enamiden und Itakonsäuremethylester

In einem Glasautoklaven wurden 6.1 mg (0.015 mmol) [Rh(cod)₂]BF₄ in 15 ml entgastem CH₂Cl₂ gelöst, 0.016 mmol Ligand und 1 mmol Substrat unter Stickstoff zugegeben und bei Raumtemperatur und 1 atm Wasserstoffdruck hydriert. Umsatz und ee wurden gaschromatographisch bestimmt.

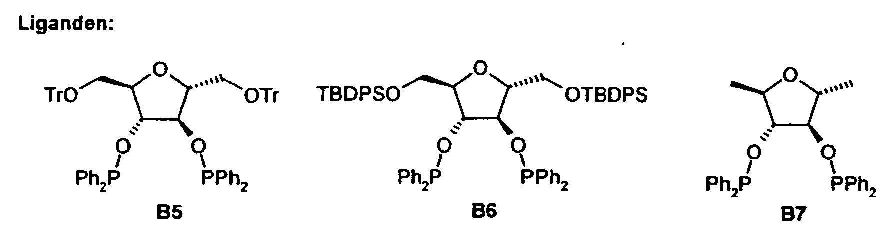

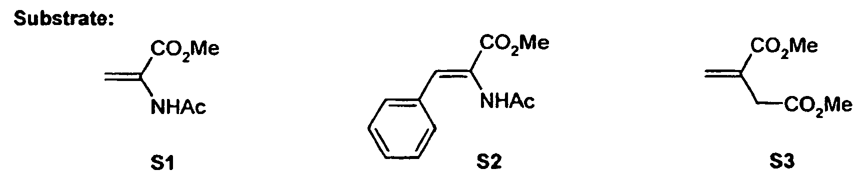

Die Ergebnisse der Hydrierungen sind in Tabelle 1 zusammengefasst.

**Tabelle 1**

| **Beispiele** | **Substrate** | **Ligand** | **Lsg. (ml)** | **t (min)** | **Subst/Rh (mol)** | **%Umsatz** | **%ee (R/S)** |
|---|---|---|---|---|---|---|---|
| 19 | S1 | B5 | CH₂Cl₂ 15 | 5 | 100 | 100 | 62(R) |
| 20 | S1 | B5 | CH₂Cl₂ 15 | 35 | 500 | 93 | 67(R) |
| 21 | S1 | B5 | CH₂Cl₂ 15 | 10 | 250 | 100 | 65(R) |
| 22 | S1 | B5 | MeOH 15 | 15 | 500 | 63 | 73(R) |
| 23 | S1 | B5 | Aceton 15 | 15 | 500 | 99 | 75(R) |
| 24 | S1 | B5 | THF 15 | 15 | 500 | 36 | 63(R) |
| 25 | S1 | B5 | Toluol/MeOH 15 | 15 | 500 | 59 | 65(R) |
| 26 | S1 | B6 | CH₂Cl₂15 | 5 | 100 | 95 | 70(R) |
| 27 | S1 | B6 | CH₂Cl₂/Aceton 2:13 | 5 | 100 | 100 | 85(R) |
| 28 | S1 | B6 | CH₂Cl₂/Aceton 2:13 | 5 | 100 | 100 | 87(R) |
| 29 | S1 | B6 | CH₂Cl₂/Aceton 2:13 | 15 | 100 | 100 | 90(R) |
| 30 | S1 | B7 | CH₂Cl₂ 15 | 5 | 100 | 100 | 73(R) |
| 31 | S1 | B7 | CH₂Cl₂/Aceton 2:13 | 5 | 100 | 100 | 79(R) |
| 32 | S2 | B5 | CH₂Cl₂15 | 10 | 100 | 91 | 59(R) |
| 33 | S2 | B5 | Aceton 15 | 10 | 100 | 91 | 73(R) |
| 34 | S2 | B6 | CH₂Cl₂15 | 5 | 100 | 98 | 73(R) |
| 35 | S2 | B6 | CH₂Cl₂/Aceton 2:13 | 5 | 100 | 96 | 80(R) |
| 36 | S2 | B7 | CH₂Cl₂ 15 | 5 | 100 | 100 | 72(R) |
| 37 | S2 | B7 | CH₂Cl₂/Aceton 2:13 | 5 | 100 | 100 | 75(R) |
| 38 | S3 | B5 | CH₂Cl₂ 15 | 15 | 100 | 100 | 48(S) |
| 39 | S3 | B5 | Aceton 15 | 60 | 100 | 33 | 35(S) |
| 40 | S3 | B6 | CH₂Cl₂ 15 | 15 | 100 | 96 | 48(S) |
| 41 | S3 | B7 | CH₂Cl₂ 15 | 5 | 100 | 96 | 53(S) |

### Herstellung von Phosphinchloriden:

### Beispiele 42-45:

**Bis-(2,4-dimethylphenyl)chlorphosphin (B42):** Eine Lösung von 2.92 ml (21.61 mmol) 4-Brom-1,3-dimethylbenzol in 3 ml Et₂O wurde bei 0°C zu einer Suspension von 0.5 g (20.56 mmol) Magnesium-Drehspänen in 7 ml THF und 7 ml Et₂O sowie einem Kristall Jod zugegeben. Die Mischung wurde auf Raumtemperatur erwärmt, über Nacht weiter gerührt und bei 0°C langsam zu einer Lösung von 1.5 ml (10.31 mmol) Et₂NPCl₂ in 8 ml THF zugetropft. Die Mischung wurde auf Raumtemperatur erwärmt und das Lösungsmittel nachfolgend im Vakuum entfernt. Nach Zugabe von 60 ml Hexan wurde die Mischung unter Argon durch Celite filtriert und eine Stunde lang mit Chlorwasserstoff versetzt. Nach Entgasung wurde der erhaltene Feststoff unter Argon abfiltriert und getrocknet. Ausbeute: 1.4 g (58.3 % d.Th.). ¹H NMR (400 MHz, CDCl₃) δ, 7.40 (d, *Jmeta*= 4.5 Hz, 2H, arom.), 7.2 (m, 4H, arom.), 2.5 (d, *J*_{*PH*}= 2.0 Hz, 6H, CH₃), 2.4 (s, 6H, CH₃). ³¹P NMR (161.974 MHz, CDCl₃) δ, 75.6.

### Beispiel 43:

**Bis-(3,5-dimethylphenyl)chlorphosphin (B43):** Dieses Produkt wurde analog zu Beispiel 42 ausgehend von 5-Brom-1,3-dimethylbenzol hergestellt. Ausbeute: 47.2 % d.Th. ¹H NMR (400 MHz, CDCl₃) δ, 7.2 (d, 4H, arom.), 7.01 (s, 2H, arom.), 2.37 (s, 12H, CH₃). ³¹P NMR (161.974 MHz, CDCl₃) δ, 83.7.

### Beispiel 44:

**Bis-(4-methoxyphenyl)chlorphosphin (B44):** Dieses Produkt wurde analog zu Beispiel 42 ausgehend von 1-Brom-4-methoxybenzol hergestellt. Ausbeute: 45 % d.Th. ¹H NMR (400 MHz, CDCl₃) δ, 7.48 (t, *Jorto*= 8.4 Hz, *J*_{*PH*}= 8.4 Hz, 4H, arom.), 6.88 (d, *Jorto*= 8.4 Hz, 4H, arom.), 3.75 (s, 6H, CH₃O). ³¹P NMR (161.974 MHz, CDCl₃) δ, 84.2.

### Beispiel 45:

**Bis-(4-Trifluoromethylphenyl)chlorphosphin (B45):** Dieses Produkt wurde analog zu Beispiel 42 ausgehend von 1-Brom-4-(trifluoromethyl)benzol hergestellt. Ausbeute: 66% d.Th. ¹H NMR (400 MHz, CDCl₃) δ, 7.33 (m, 8H, arom.). ³¹P NMR (161.9 MHz, CDCl₃) δ, 76.3.

### Herstellung von Aminophosphinen:

### Beispiel 46:

**(Diethylamino)-bis(2,4-dimethylphenyl)phosphin (B46):** Eine Lösung von 2.92 ml (21.61 mmol) 4-Brom-1,3-dimethylbenzol in 3 ml Et₂O wurde bei 0°C zu einer Suspension von 0.5 g (20.56 mmol) Magnesium-Drehspänen in 7 ml THF und 7 ml Et₂O sowie einem Kristall Jod zugegeben. Die Mischung wurde auf Raumtemperatur erwärmt, über Nacht weiter gerührt und bei 0°C langsam zu einer Lösung von 1.5 ml (10.31 mmol) Et₂NPCl₂ in 8 ml THF zugetropft. Die Mischung wurde auf 5-10°C erwärmt, zwei Stunden weiter gerührt und das Lösungsmittel nachfolgend im Vakuum entfernt. Nach Zugabe von 60 ml Hexan wurde die Mischung unter Argon durch Celite filtriert und das Lösungsmittel nachfolgend im Vakuum entfernt. Ausbeute: 1.37 g (59 % d.Th.). ¹H NMR (400 MHz, CDCl₃) δ, 6.9 (m, 6H), 3.03 (m, 4H, CH₂), 2.20 (s, 12H, CH₃), 0.81 (t, ³*J*=7.0 Hz, 6H, CH₃) ³¹P NMR (161.9 MHz, CDCl₃) δ, 47.8.

### Beispiel 47:

**(Diethylamino)-bis(3,5-dimethylphenyl)phosphin (B47):** Dieses Produkt wurde analog zu Beispiel 46 ausgehend von 5-Brom-1,3-dimethylbenzol hergestellt. Ausbeute: 56.8% d.Th. ¹H NMR (400 MHz, CDCl₃) δ, 6.90 (d, 4H, arom.), 6.76 (s, 2H, arom.), 2.95 (m, 4H, CH₂), 2.11 (s, 12H, CH₃), 0.80 (t, ^{*3*}*J*=7.0 Hz, 6H, CH₃). ³¹P NMR (161.9 MHz, CDCl₃) δ, 61.6.

### Beispiel 48:

**(Diethylamino)-bis(4-methoxyphenyl)phosphin (B48):** Dieses Produkt wurde analog zu Beispiel 46 ausgehend von 1-Brom-4-methoxybenzol hergestellt. Ausbeute: 57.2% d.Th. ¹H NMR (400 MHz, CDCl₃) δ, 7.48 (dd, *Jorto*= 8.8 Hz, *J*_{*PH*}= 6.4 Hz, 4H, arom.), 6.88 (m, 4H, arom.), 3.81 (s, 6H, CH₃O), 3.06 (q, ³*J*=7.2 Hz, 2H, CH₂), 0.96 (t, ^{*3*}*J=7.2* Hz, 3H, CH₃). ³¹P NMR (161.9 MHz, CDCl₃) δ, 59.5.

### Beispiel 49:

**(Diethylamino)-bis(4-trifluoromethylphenyl)phosphin (B49): Dieses Produkt** wurde analog zu Beispiel 46 ausgehend von 1-Brom-4-(trifluoromethyl)benzol hergestellt. Ausbeute: 61.4 % d.Th. ¹H NMR (400 MHz, CDCl₃) δ, 7.39 (m, 8H, arom.), 2.92 (m, 4H, CH₂), 0.82 (t, ³*J*=7.0 Hz, 3H, CH₃). ³¹P NMR (161.9 MHz, CDCl₃) δ, 61.5.

### Herstellung von Biphosphorverbindungen:

### Beispiel 50:

### 2,3-bis-O-(Di(4-methoxyphenyl)phosphino)-1,6-di-O-(tert-butyldiphenylsilyl)-2,5-anhydro-D-mannit (B50):

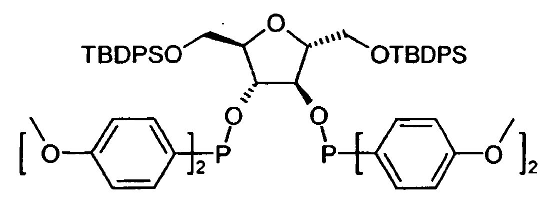

Eine Mischung von 100 mg (0.156 mmol) 1,6-Di-O-(tert-Butyldiphenylsilyl-2,5-anhydro-D-mannit **(B2)** und 109 mg (0.343 mmol) Diethylamino-bis(p-methoxybenzol)phosphin **(B48)** in 2.2 ml wasserfreiem Toluol wurde bei 112°C über Nacht gerührt. Nach Abkühlen wurde das Lösungsmittel im Vakuum entfernt und das Rohprodukt mittels Säulenchromatographie gereinigt. Ausbeute: 40 mg (22.7% d.Th.). ¹H NMR (400 MHz, C₆D₆) δ, 7.93 (m, 8H, arom.), 7.67 (m, 8H, arom.), 7.26 (m, 12H, arom.), 6.84 (m, 8H, arom.), 5.38 (dd, *J*=7.9 Hz, *J*=4 Hz, 2H, CH), 4.59 (m, 2H, CH), 4.09 (dd, *J*=10.9 Hz, *J*=4.2 Hz, 2H, CH₂), 3.99 (dd, *J*=10.9 Hz, *J*=4.2 Hz, 2H, CH₂), 3.37 (s, 6H, CH₃O), 3.35 (s, 6H, CH₃O), 1.32 (s, 9H, CH₃), 1.27 (s, 9H, CH₃). ³¹P NMR (161.9 MHz, C₆D₆) δ, 116.3.

### Beispiel 51:

### 2,3-bis-O-(Di((4-Trifluoromethyl)phenyl)phosphino)-1,6-di-O-(tert-butyldiphenylsilyl)-2,5-anhydro-D-mannit (B51):

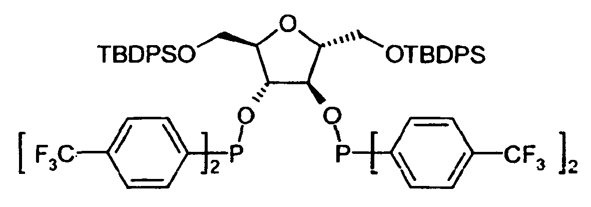

Dieses Produkt wurde analog zu Beispiel 50 ausgehend von 1,6-Di-O-(tert-Butyldiphenylsilyl-2,5-anhydro-D-mannit **(B2)** und (Diethylamino)-bis(4-trifluoromethylphenyl)phosphin **(B49)** hergestellt. Ausbeute: 40 mg (28% d.Th.). ¹H NMR (400 MHz, C₆D₆) δ, 7.9 (m, 2H, arom.), 7.85 (m, 4H, arom.), 7.74 (m, 2H, arom.), 7.40-7.29 (m, 26H, arom.), 7.0 (m, 2H, arom), 5.36 (m, 2H, CH), 4.41 (m, 2H, CH), 4.02 (dd, *J*=11.4 Hz, *J*=3.5 Hz, 2H, CH₂), 3.79 (dd, *J*=11.4 Hz, *J*=3.5 Hz, 2H, CH₂), 1.28 (s, 9H, CH₃). ³¹P NMR δ, (161.9 MHz, C₆D₆) 111.5.

### Beispiel 52:

### 2-O-(Di(2,4-dimethylphenyl)phosphino)-1,6-di-O-(tert-butyldiphenylsilyl)-2,5-anhydro-D-mannit (B52):

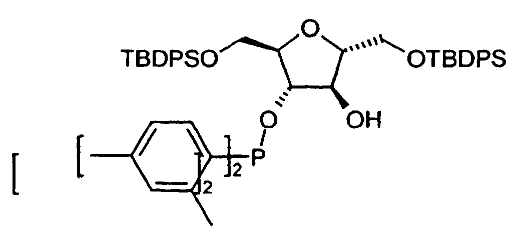

Zu einer Lösung von 300 mg (0.468 mmol) 1,6-di-*O*-(*tert*-butyldiphenylsilyl)-2,5-anhydro-D-mannit **(B2)** und 0.26 ml wasserfreiem Et₃N (1.86 mmol) wurde eine Lösung von 337 mg (1.219 mmols) bis-(2,4-dimethylphenyl)-chlorphosphin **(B46)** in 2 ml wasserfreiem THF zugegeben und bei Raumtemperatur über Nacht gerührt. Nach Zugabe von Ethylether wurde die Mischung durch Celite filtriert, das Lösungsmittel im Vakuum entfernt und das Rohprodukt wird mittels Säulenchromatographie gereinigt. Ausbeute 180 mg (45% d.Th.). ¹H NMR (400 MHz, CDCl₃) δ, 7.59-6.87 (m, 26H, arom.), 4.47 (m, 1H, CH), 4.31 (m, 1H, CH), 3.99 (m, 2H, CH), 3.69 (m, 3H, CH₂), 3.54 (dd, 1H, CH₂), 2.79 (s, OH), 2.30 (s, 3H, CH₃), 2.17 (s, 3H, CH₃), 2.11 (s, 3H, CH₃), 2.06 (s, 3H, CH₃), 0.96 (s, 9H, CH₃), 0.94 (s, 9H, CH₃). ¹³C NMR (75.4 MHz, CDCl₃) δ, 138.1-127.6 (CH, C, arom.), 86.0 (^{*2*}*J*_{*C-P*}=18 Hz, CH), 84.9 (CH), 83.9 *(*^{*3*}*J*_{*C-P*}=6.13 Hz, CH), 78.0 (^{*2*}*J*_{*C-P*}=4.5 Hz, CH CH), 64.7 (CH₂), 64.1 (CH₂), 27.1 (CH₃), 27.0 (CH₃), 21.4 (C), 20.5 (d, ³*J*=48.4 Hz, CH₃), 20.3 (d, ^{*3*}*J*=48.4 Hz, CH₃), 19.6 (s, CH₃), 19.5 (s, CH₃). ³¹P NMR (161.9 MHz, CDCl₃) δ, 102.9.

### Beispiel 53:

### 2-O-(2,4-dimethylphenylphosphino)-3-O-(diphenylphosphino)-1,6-di-O-(tert-butyldiphenylsilyl)-2,5-anhydro-D-mannit (B53):

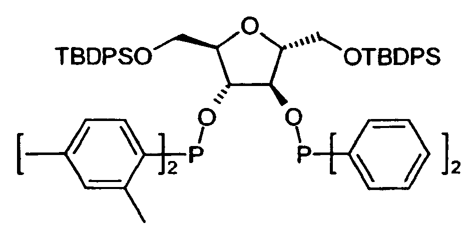

Zu einer Lösung von 58 mg (0,06 mmol) 2-O-(2,4-dimethylphenylphosphino)-1,6-dideoxy-2,5-anhydro-D-mannit **(B52)** und 0.032 ml (0.23 mmol) wasserfreiem Et₃N in 0.5 ml wasserfrei THF wurde eine Lösung von 0.0125 ml (0.066 mmol) Chlordiphenylphosphin zugegeben. Die Mischung wurde auf Raumtemperatur erwärmt und über Nacht weiter gerührt. Nach Zugabe von entgastem, wasserfreiem Hexan wurde die Mischung durch Celite filtriert, das Lösungsmittel im Vakuum entfernt und das Rohprodukt mittels Säulenchromatographie gereinigt. Ausbeute 29.4 mg (45.9% d.Th.). ¹H NMR (400 MHz, CDCl₃) δ, 7.67-6.91 (m, 36H, arom.), 4.8 (m, 2H, CHx2), 4.15 (m, 2H, CHx2), 3.73 (m, 2H, CH₂), 3.59 (m, 2H, CH₂), 2.25 (s, 3H, CH₃), 2.23 (s, 3H, CH₃), 2.19 (s, 3H, CH3), 2.14 (s, 3H, CH₃), 1.1 (s, 9H, CH₃), 1 (d, 9H, CH₃). ³¹P NMR (161.9 MHz, C₆D₆) δ, 114.1, 102.7.

### Beispiel 54:

### 2-O-(2,4-Dimethylphenylphosphino)-3-O-(4,8-di-tert.-butyl-2,10-dimethyl-12H-dibenzo[δ,γ][1,3,2]dioxaphosphocino)-1,6-di-O-(tert-butyldiphenyisilyl)-2,5-anhydro-D-mannit (B54):

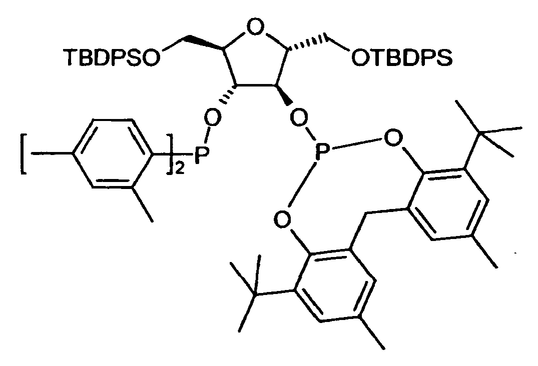

Zu einer Lösung von 178 mg (0.202 mmol) 2-*O*-(2,4-dimethylphenylphosphino)-1,6-di-*O*-(*tert*-butyldiphenylsilyl)-2,5-anhydro-D-mannit **(B52)** und 0.100 ml (1.23 mmol) wasserfreiem Pyridin in 1 ml wasserfreiem Toluol wurde bei 0°C eine Lösung von 100 mg (0.52 mmol) 4,8-Di-tert.-butyl-6-chlor-2,10-dimethyl-12h-dibenzo[δ,γ]-[1,3,2]dioxaphosphocin und 0.100 ml (1.23 mmol) wasserfreies Pyridin in 1 ml wasserfreiem Toluol getropft. Die Mischung wurde auf Raumtemperatur erwärmt und über Nacht weiter gerührt. Nach Zugabe von entgastem, wasserfreiem Hexan wurde die Mischung durch Celite filtriert, das Lösungsmittel im Vakuum entfernt und das Rohprodukt mittels Säulenchromatographie gereinigt. Ausbeute 100 mg (39.6% d.Th.). ¹H NMR (400 MHz, C₆D₆) δ, 7.97-6.38 (m, 30H, arom.), 5.50 (m, 1H, CH), 5.21 (m, 1H, CH), 4.83 (m, 1H, CH), 4.53 (d, *J*=10.4 Hz, 1 H, CH₂), 4.50 (m, 3H, CH, CH₂), 4.39 (dd, 1H, *J*=10.8 Hz, *J*=5.59 Hz, CH₂), 4.15 (dd, 1H, *J*=10.8 Hz, *J*=5.59 Hz, CH₂), 3.3 (d, *J*=10.4 Hz,1H, CH₂), 2.59 (s, 3H, CH₃), 2.51 (s, 3H, CH3), 2.15 (s, 3H, CH₃), 2.12 (s, 6H, CH₃), 2.1 (s, 3H, CH₃), 1.54 (s, 9H, CH₃), 1.53 (s, 9H, CH₃), 1.36 (s, 9H, CH₃), 1.32 (s, 9H, CH₃). ³¹P NMR (161.9 MHz, C₆D₆) δ, 128.8,103.6.

### Beispiel 55:

### 2-O-(2,4-Dimethylphenylphosphino)-3-O-(2,10-dimethyl-4,8-bis(1-methylcyclohexyl)-12H-dibenzo[δ,γ][1,3,2]dioxaphosphocino)-1,6-di-O-(tert-butyldiphenylsilyl)-2,5-anhydro-D-mannit (B55):

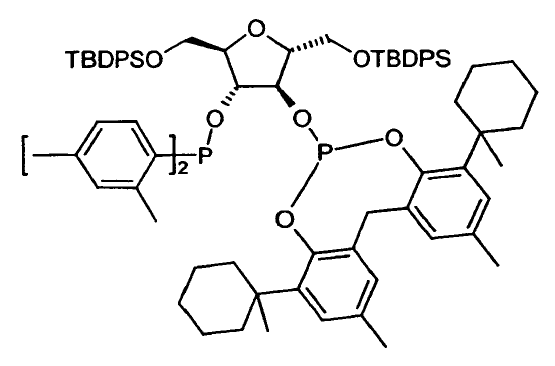

Dieses Produkt wurde analog zu Beispiel 54 ausgehend von 2-*O*-(2,4-dimethylphenylphosphino)-1,6-di-*O*-(*tert*-butyldiphenylsilyl)-2,5-anhydro-D-mannit **(B52)** und 6-Chlor-2,10-dimethyl-4,8-bis(1-methylcyclohexyl)-12H-dibenzo[δ,γ][1,3,2]-dioxaphosphocin hergestellt. Ausbeute 71 mg (32.4% d.Th.). ¹H NMR (400 MHz, C₆D₆) δ, 7.99-6.3 (m, 30H, arom.), 5.44 (m, 1H, CH), 5.14 (m, 1H, CH), 4.81 (m, 1H, CH), 4.57 (dd, *J*=10.7 Hz, *J*=4.5 Hz, 1H, CH₂), 4.53 (m, 1H, CH, CH₂), 4.41 (dd, 1 H, *J*=10.7 Hz, *J*=4.5 Hz, CH₂), 4.29 (dd, 1H, *J*=10.8 Hz, *J*=5.0 Hz, CH₂), 4.18 (dd, 1H, *J*=10.8 Hz, *J*=5.0 Hz, CH₂), 3.3 (d, *J*=12.7 Hz, 1H, CH₂), 2.59 (s, 3H, CH₃), 2.52 (s, 3H, CH3), 2.24 (s, 3H, CH₃), 2.22 (s, 3H, CH₃), 2.18 (s, 3H, CH₃), 2.17 (s, 3H, CH₃), 1.68-1.57 (m, CH₂), 1.48 (s, 3H, CH₃), 1.47 (s, 3H, CH₃), 1.36 (s, 9H, CH₃), 1.33 (s, 9H, CH₃). ³¹P NMR (161.9 MHz, CDCl₃) δ, 128.7, 105.3.

### Iridium-katalysierte Hydrogenierung von Iminen und Enamiden

### Beispiele 56-78:

0.01 Moläquivalente Übergangsmetallverbindung und 0.012 Moläquivalente Ligand wurden unter Argon in entgastem CH₂Cl₂ gelöst (0.015 M) und bei Raumtemperatur 1/2 Stunde gerührt. Nach Zugabe von einem Moläquivalent Substrat in entgastem CH₂Cl₂ (0.15 M) unter Argon wurde die erhaltene Mischung in einem Autoklaven bei entsprechender Temperatur unter Wasserstoffdruck hydriert. Umsatz und ee wurden chromatographisch bestimmt.

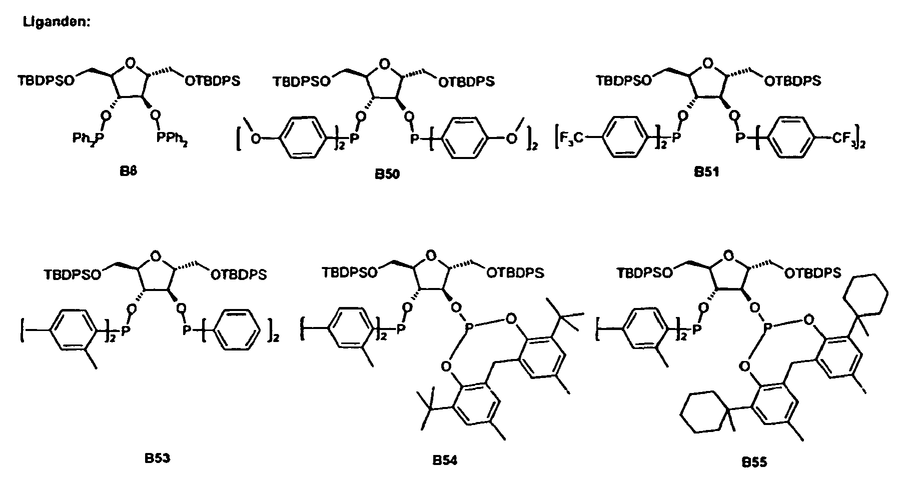

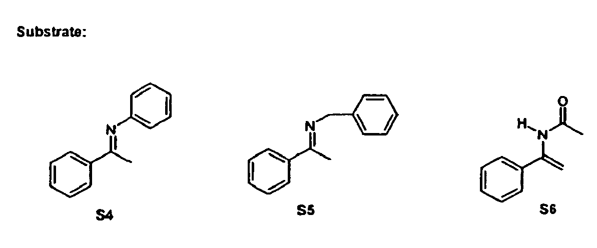

Die Ergebnisse der Hydrierungen sind in Tabelle 2 zusammengefasst.

**Tabelle 2**

| **Beispiele** | **Substrate** | **Ligand** | **Metallprecursor** | **Additiv** | **T (°C)** | **P (bar)** | **Zeit (h)** | **Substrat/ Metall (mol)** | **Umsatz (%)** | **ee (%)** |
|---|---|---|---|---|---|---|---|---|---|---|
| 56 | S4 | B6 | [Ir(cod)₂]BF₄ | - | 25 | 70 | 16 | 100 | 100 | 65 |
| 57 | S4 | B51 | [Ir(cod)₂]BF₄ | - | 25 | 70 | 16 | 100 | 100 | 31 |
| 58 | S4 | B50 | [Ir(cod)₂]BF₄ | - | 25 | 70 | 16 | 100 | 97 | 71 |
| 59 | S4 | B50 | [Ir(cod)₂]BF₄ | 4% Phthalimid | 25 | 70 | 16 | 100 | 99 | 70 |
| 60 | S4 | B50 | [Ir(cod)₂]BF₄ | 4% BzNH₂ | 25 | 70 | 16 | 100 | 41 | 52 |
| 61 | S5 | B54 | [Ir(cod)₂]BF₄ | - | 25 | 70 | 16 | 100 | 86 | 73 |
| Beispiele | Substrate | Lgrand | Metallprecusor | Additiv | T (°C) | P (bar) | Zeit (h) | Substrat/Metall(moi) | Umsatz (%) | ee (%) |
| 62 | S5 | B54 | [Ir(cod)₂]BF₄ | - | 25 | 70 | 0.5 | 100 | 24 | 68 |
| 63 | S5 | B54 | [Ir(cod)₂]BF₄ | - | 0 | 70 | 2 | 100 | 4 | 40 |
| 64 | S5 | B54 | [Ir(cod)₂]BF₄ | 10%12 | 0 | 70 | 2 | 100 | 1 | 76 |
| 65 | S5 | B54 | [Ir(cod)₂]BF₄ | - | 50 | 70 | 0.5 | 100 | 80 | 61 |
| 66 | S5 | B54 | [Ir(cod)₂]BF₄ | 10% Phthalimid | 25 | 70 | 16 | 100 | 89 | 75 |
| 67 | S5 | B54 | [Ir(cod)₂]BF₄ | 10% BzNH₂ | 25 | 70 | 16 | 100 | 91 | 75 |
| 68 | S5 | B55 | [Ir(cod)₂]BF₄ | 10% Phthalimid | 25 | 70 | 16 | 100 | 63 | 54 |
| 69 | S5 | B55 | [Ir(cod)₂]BF₄ | 10% BzNH₂ | 25 | 70 | 16 | 100 | 81 | 58 |
| 70 | S6 | B6 | [Rh(nbd)₂]PF₆ | - | 25 | 3.5 | 24 | 100 | 100 | 48 |
| 71 | S6 | B51 | [Rh(nbd)₂]PF₆ | - | 25 | 3.5 | 24 | 100 | 99 | 6 |
| 72 | S6 | B50 | [Rh(nbd)₂]PF₆ | - | 25 | 3.5 | 24 | 100 | 100 | 33 |
| 73 | S6 | B53 | [Rh(nbd)₂]PF₆ | - | 25 | 3.5 | 24 | 100 | 98 | 20 |
| 74 | S6 | B54 | [Rh(nbd)₂]PF₆ | - | 25 | 3.5 | 24 | 100 | 97 | 22 |
| 75 | S6 | B6 | [Rh(nbd)₂]PF₆ | - | 25 | 3.5 | 24 | 100 | 56 | 40 |
| 76 | S6 | B51 | [Rh(nbd)₂]PF₆ | - | 25 | 3.5 | 24 | 100 | 89 | 50 |
| 77 | S6 | B50 | [Rh(nbd)₂]PF₆ | - | 25 | 3.5 | 24 | 100 | 89 | 28 |
| 78 | S6 | B54 | [Rh(cod)₂]BF₄ | - | 25 | 3.5 | 24 | 100 | 98 | 27 |

### Beispiel 79

In einem Autoklaven wurden 28.1 mg (0.022 mmol) [Ir(cod)(B6)]BF₄ **(B18)** und 0.39 g (2 mmol) *N*-(Phenylethylidene)anilin **(S4)** im 10 ml entgastem CH₂Cl₂ gelöst. Die Mischung wurde bei Raumtemperatur und 50 bar Wassestoffdruck hydriert. Umsatz und ee wurden gaschromatographisch bestimmt. 99% Umsatz, 67% ee.

## Patentansprüche

1. Verbindungen der Formel (I), 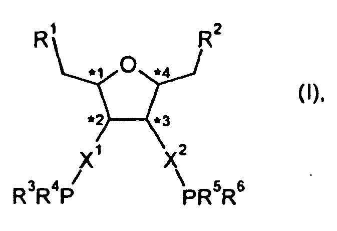 in der
• *1, *2, *3 und *4 jeweils unabhängig voneinander ein stereogenes Kohlenstoffatom markieren, das in R- oder S- Konfiguration vorliegt,
• X¹ und X² jeweils unabhängig voneinander fehlen oder für Sauerstoff stehen und
• R¹ und R² jeweils unabhängig voneinander stehen können für: Wasserstoff, C₁-C₂₀-Alkyl, C₁-C₂₀-Fluoralkyl, C₂-C₂₀-Alkenyl, C₄₋C₂₄-Aryl, C₅-C₂₅-Arylalkyl, C₆-C₂₆-Arylalkenyl oder NR⁷R⁸, OR⁸, -(C₁-C₈-Alkyl)-OR⁸, -(C₁-C₈-Alkyl)-NR⁷R⁸ oder -O₂CR⁸, wobei R⁷ und R⁸ jeweils unabhängig voneinander für C₁-C₈-Alkyl, C₅-C₁₄₋Arylalkyl oder C₄-C₁₅-Aryl stehen oder R⁷ und R⁸ zusammmen für einen cyclischen Aminorest mit insgesamt 4 bis 20 Kohlenstoffatomen steht,
oder R¹ und R² jeweils unabhängig voneinander für Reste der Formel (II) stehen
-R⁹-SiR¹⁰R¹¹R¹² (II)
in der
R⁹ fehlt, für Sauerstoff oder Methylen steht und
R¹⁰, R¹¹ und R¹² jeweils unabhängig voneinander für C₁-C₁₂-Alkyl, C₅-C₁₅-Arylalkyl oder C₄-C₁₄-Aryl stehen und
• R³, R⁴, R⁵ und R⁶ jeweils unabhängig voneinander für R¹³, OR¹⁴ oder NR¹⁵R¹⁶ stehen können, wobei R¹³, R¹⁴, R¹⁵ und R¹⁶ jeweils unabhängig für C₁-C₁₂-Alkyl, C₅-C₁₅-Arylalkyl oder C₄-C₁₄-Aryl stehen oder NR¹⁵R¹⁶ zusammen für einen cyclischen Aminorest mit 4 bis 20 Kohlenstoffatomen steht oder R³ und R⁴ bzw. R⁵ und R⁶ jeweils zusammen für -O-R¹⁷-O- stehen, wobei R¹⁷ für Reste steht, die ausgewählt sind aus der Gruppe C₂-C₄-Alkylen, 1,2-Phenylen, 1,3-Phenylen, 1,2-Cyclohexylen, 1,1'-Ferrocenylen, 1,2-Ferrocenylen, 2,2'-(1,1'-Binaphtylen), 2,2'-(1,1')-Biphenylen und 1,1'-(Diphenyl-2,2'-methylen)-diyl, wobei die genannten Reste gegebenenfalls einfach oder mehrfach durch Reste substituiert sein können, die ausgewählt sind aus der Gruppe Fluor, Chlor, C₁-C₈-Alkoxy und C₁-C₈₋Alkyl.

2. Verbindungen gemäß Anspruch 1, wobei in Formel (I) *¹,*²,*³,*⁴ zusammen folgende Stereoisomere des zentralen substituierten Furanringes definieren:
(1R,2R,3R,4R), (1R,2R,3R,4S), (1R,2R,3S,4S), (1R,2S,3S,4S), (1R,2S,3R,4S), (1R,2S,3S,4R), (1R,2R,3S,4R), (1S,2S,3R,4S), (1S,2S,3S,4S), (1S,2S,3S,4R), (1S,2S,3R,4R), (1S,2R,3R,4R), (1S,2R,3S,4R), (1S,2R,3R,4S), (1S,2S,3R,4S), (1R,2R,3S,4R).

3. Verbindungen gemäß mindestens einem der Ansprüche 1 bis 2, **dadurch gekennzeichnet, dass** in Formel (I) R¹ und R² jeweils unabhängig voneinander für Wasserstoff, C₁-C₄-Alkyl, C₄-C₁₄-Aryl, O-R⁸, O₂C-R⁸, wobei R⁸ vorzugsweise für C₁-C₁₂-Alkyl, C₅-C₂₅-Arylalkyl oder C₄-C₁₄-Aryl steht, oder OSiR¹⁰R¹¹R¹², wobei R¹⁰, R¹¹, und R¹² vorzugsweise jeweils unabhängig für C₁-C₁₂-Alkyl oder C₄-C₁₄-Aryl stehen.

4. Verbindungen gemäß mindestens einem der Ansprüche 1 bis 3, **dadurch gekennzeichnet, dass** in Formel (I) R¹ und R² jeweils unabhängig voneinander für Wasserstoff, tert.-Butoxy, Trityloxy, tert-Butyldimethylsilyloxy, tert-Butyldiphenylsilyloxy, Trimethylsilyloxy, Triethylsilyloxy, Triisopropylsilyloxy, neo-Pentoxy oder 1-Adamantoxy stehen.

5. Verbindungen gemäß mindestens einem der Ansprüche 1 bis 4, **dadurch gekennzeichnet, dass** in Formel (I) R³, R⁴, R⁵ und R⁶ jeweils unabhängig voneinander für R¹³, OR¹⁴ oder NR¹⁵R¹⁶ stehen, wobei R¹³, R¹⁴, R¹⁵ und R¹⁶ jeweils unabhängig für C₁-C₁₂-Alkyl oder C₄-C₁₄-Aryl stehen oder NR¹⁵R¹⁶ zusammen für einen cyclischen Aminorest mit 4 bis 12 Kohlenstoffatomen oder R³ und R⁴ bzw. R⁵ und R⁶ jeweils zusammen für -O-R¹⁷-O- stehen, wobei R¹⁷ für Ethylen, 1,2-Phenylen, 1,3-Phenylen, 1,2-Cyclohexylen, 1,1'-Ferrocenylen, 1,2-Ferrocenylen, zwei- oder vierfach durch C₁-C₈-Alkyl substituiertes 1,1'-(Diphenyl-2,2'-methylen)-diyl, 2,2'-(1,1'-Binaphtylen) oder 2,2'-(1,1')-Biphenylen steht, wobei 2,2'-(1,1'-Binaphtylen) oder 2,2'-(1,1')-Biphenylen zumindest in 6,6'-Position durch Reste substituiert ist, die ausgewählt sind aus der Gruppe C₁-C₈-Alkoxy und C₁-C₈-Alkyl und weiterhin in 5,5'-,4,4'-, 3,3'- oder 2,2'-Position durch Reste substituiert sein kann, die ausgewählt sind aus der Gruppe Fluor, Chlor, C₁-C₈-Alkoxy und C₁-C₈-Alkyl.

6. Verbindungen gemäß mindestens einem der Ansprüche 1 bis 5, **dadurch gekennzeichnet, dass** in Formel (I) R³, R⁴, R⁵ und R⁶ jeweils unabhängig voneinander für R¹³, OR¹⁴ oder NR¹⁵R¹⁶, wobei R¹³ und R¹⁴ jeweils unabhängig steht für Methyl, Ethyl, n-Propyl, iso-Propyl, tert-Butyl, Cyclohexyl, Phenyl, 2-(C₁-C₈)-Alkylphenyl, 3-(C₁-C₈)-Alkylphenyl, 4-(C₁₋C₈)-Alkylphenyl, 2,6-Di-(C₁-C₈)-alkylphenyl, 3,5-Di-(C₁-C₈)-alkylphenyl, 2,4-Di-(C₁-C₈)-alkylphenyl, 3,4,5-Tri-(C₁-C₈)-alkylphenyl, 2-(C₁-C₈)-Alkoxyphenyl, 3-(C₁-C₈)-Alkoxyphenyl, 4-(C₁-C₈)-Alkoxyphenyl, 2,4-Di-(C₁-C₈)-alkoxyphenyl, 2,6-Di-(C₁-C₈)-alkoxyphenyl, 3,5-Di-(C₁-C₈)-alkoxyphenyl, 3,4,5-Tri-(C₁-C₈)-alkoxyphenyl, 3,5-Dialkyl-4-(C₁-C₈)-alkoxyphenyl, 3,5-(C₁-C₈)-Dialkyl-4-di-(C₁-C₈)-alkylaminophenyl, 4-Di-(C₁₋C₈)-alkylaminophenyl, 3,5-Bis-((C₁-C₄)-fluoralkyl), 2,4-Bis-((C₁-C₄)-fluoralkyl)phenyl, 4-((C₁-C₄)-Fluoralkyl)phenyl und ein-, zwei- drei- oder vierfach durch Fluor und/oder Chlor substituiertes Phenyl, Fluorenyl oder Naphthyl oder NR¹⁵R¹⁶ als Ganzes für Dimethylamino, Diethylamino, Pyrrolidino oder Diisopropylamino steht oder R³ und R⁴ beziehungsweise R⁵ und R⁶ jeweils paarweise für O-R¹⁷-O stehen, wobei R¹⁷ für 1,1'-Bis-(4,6-di-(C₁-C₈-Alkyl)-phenyl)-2,2'-methylen)-diyl steht oder wobei R¹⁷ für (R)-1,1'-Biphenyl-2,2'-diyl, (S)-1,1'-Biphenyl-2,2'-diyl, (R)-1,1'-Binaphthyl-2,2'-diyl, (S)-1,1'-Binaphthyl-2,2'-diyl, 1,1'-[Bis-(4-methyl-6-tert.-butyl-phenyl)-2,2'-methylen)]-diyl oder 1,1'-[Bis-(4-methyl-6-(1-methylcyclohexyl)-2,2'-methylen)]-diyl steht.

7. Verbindungen gemäß mindestens einem der Ansprüche 1 bis 6, **dadurch gekennzeichnet, dass** in Formel (I) R³ und R⁴ bzw. R⁵ und R⁶ paarweise identisch sind.

8. Verbindungen gemäß mindestens einem der Ansprüche 1 bis 2, **dadurch gekennzeichnet, dass** sie den Formel (Ia) bis (Ii) gehorchen 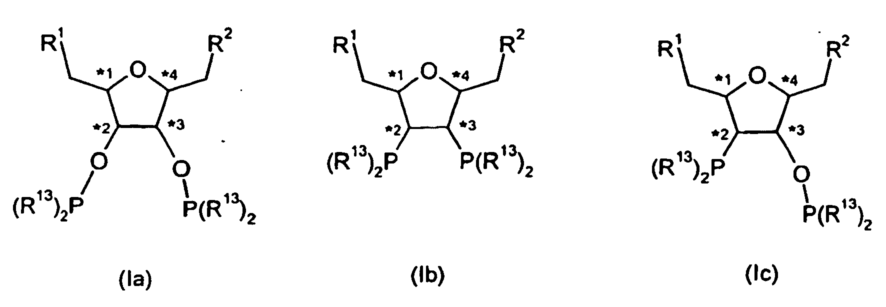 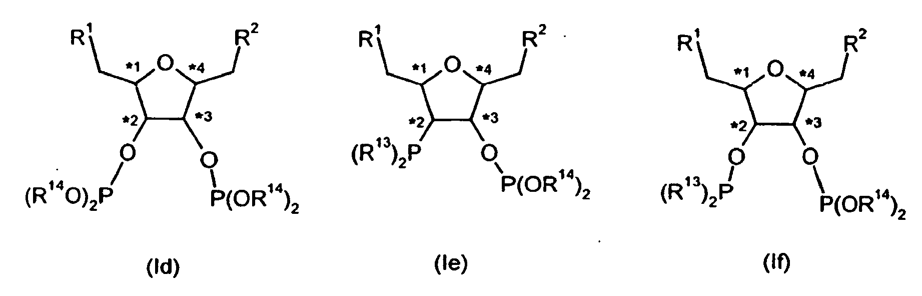 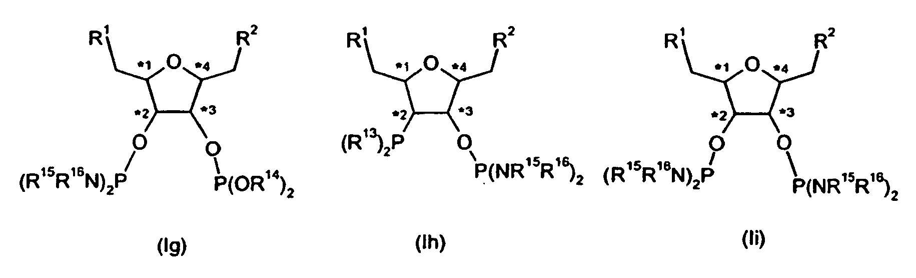 in denen *¹,*²,*³,*⁴, R¹, R², R¹³, R¹⁴, R¹⁵ und R¹⁶ jeweils die unter der Formel (I) in Anspruch 1 genannte Bedeutung besitzen.

9. Verbindungen der Formel (XIII), 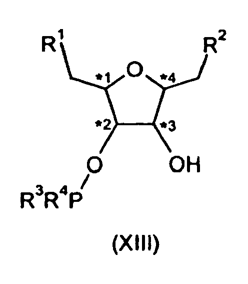 in der R¹, R², R³ und R⁴ die gleiche Bedeutung besitzen, die unter der Formel (I) in Anspruch 1 angegeben ist.

10. 2,3-bis-*O*-(Diphenylphosphino)-1,6-di-*O*-(triphenylmethyl)-2,5-anhydro-D-mannit, 2,3-bis-*O*-(Diphenylphosphino)-1,6-dideoxy-2,5-anhydro-D-mannit, 2,3-bis-*O*-(Diphenylphosphino)-1,6-di-*O*-(*tert*-butyldiphenylsilyl)-2,5-anhydro-D-mannit, 2,3-bis-*O*-(Diphenylphosphino)-1,6-di-*O*-(triphenylmethyl)-2,5-anhydro-L-iditol, 2,3-bis-*O*-(Diphenylphosphino)-1,6-di-*O*-(*tert*-butyldiphenylsilyl)-2,5-anhydro-L-iditol, 2,3-bis-*O*-(Diphenylphosphino)-1,6-dideoxy-2,5-anhydro-L-iditol, 2,3-bis-*O*-(Di(4-Methoxyphenyl)phosphino)-1,6-di-*O*-(*tert*-butyldiphenylsilyl)-2,5-anhydro-D-mannit, 2,3-bis-*O*-(Di((4-Trifluoromethyl)phenyl)phosphino)-1,6-di-*O*-(*tert*-butyldiphenylsilyl)-2,5-anhydro-D-mannit, 2-*O*-(Di(2,4-Dimethylphenyl)phosphino)-3-*O-*(diphenylphosphino)-1,6-di-*O*-(*tert*-butyldiphenylsilyl)-2,5-anhydro-D-mannit, 2-*O*-(Di(2,4-Dimethylphenyl)phosphino)-3-*O*-(4,8-ditert-butyl-2,10-dimethyl-12H-dibenzo[δ,γ][1,3,2]dioxaphosphocino)-1,6-di-*O*-(*tert*-butyldiphenylsilyl)-2,5-anhydro-D-mannit und 2-*O*-(Di(2,4-Dimethylphenyl)phosphino)-3-*O*-(2,10-dimethyl-4,8-bis(1-methylcyclohexyl)-12H-dibenzo[δ,γ]-[1,3,2]dioxaphosphocino)-1,6-di-*O*-(*tert*-butyldiphenylsilyl)-2,5-anhydro-D-mannit.

11. Verfahren zur Herstellung von Verbindungen der Formel (XV) 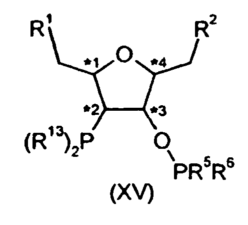 in der R¹, R², R⁵, R⁶ und R¹³ die unter der Formel (I) in Anspruch 1 angegebenen Bedeutungen besitzen, **dadurch gekennzeichnet, dass**
im Schritt a)
Verbindungen der Formel (XVI) 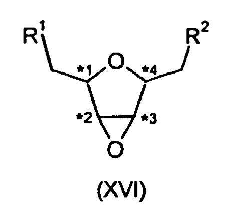 in der R¹ und R² die unter der Formel (I) in Anspruch 1 genannte Bedeutung besitzen in Gegenwart von Verbindungen der Formel (XVII)
(R¹³)₂PMet² (XVII)
in der
Met² für Lithium, Natrium oder Kalium steht und
R¹³ die unter der Formel (I) in Anspruch 1 genannte Bedeutung besitzt
zu Verbindungen der Formel (XVIII) umgesetzt werden 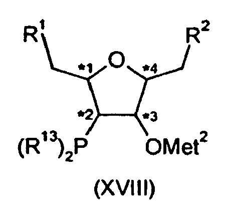 in der R¹, R², Met² und R¹³ die vorstehend genannte Bedeutung besitzen
und im Schritt b)
die Verbindungen der Formel (XVIII) mit Verbindungen der Formel (XIIb)
R⁵R⁶P-Y (XIIb)
in der R⁵ und R⁶ die gleiche Bedeutung besitzen die unter der Formel (I) in Anspruch 1 angegeben sind und
Y für Chlor, Brom, Iod, Dimethylamino oder Diethylamino zu Verbindungen der Formel (XV) umsetzt.

12. Verfahren gemäß Anspruch 11, **dadurch gekennzeichnet, dass** die Verbindungen der Formel (XVII) durch Ansäuern in Verbindungen der Formel (XIX) überführt werden 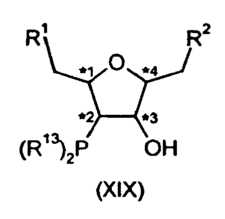 und im Schritt b) durch Umsetzung mit Verbindungen der Formel (XIIb) in Verbindungen der Formel (XV) überführt werden.

13. Verfahren gemäß Anspruch 12, **dadurch gekennzeichnet, dass** Schritt b) in Gegenwart einer Base durchgeführt wird.

14. Verbindungen der Formel (XVIII) 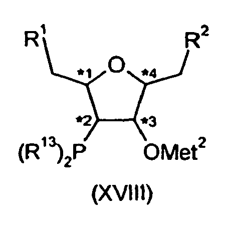 in der R¹, R² und R¹³ die unter der Formel (I) im Anspruch 1 und Met² die unter der Formel (XVII) im Anspruch 10 genannte Bedeutung besitzen.

15. Verbindungen der Formel (XIX) 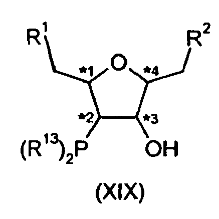 in der R¹, R² und R¹³ die unter der Formel (I) im Anspruch 1 genannte Bedeutung besitzen.

16. Verbindungen der Formel (XXa) 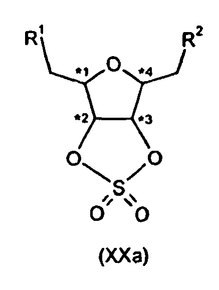 in denen R¹ und R² die unter der Formel (I) in Anspruch 1 genannte Bedeutung besitzen.

17. Verbindungen der Formel (XXIa), 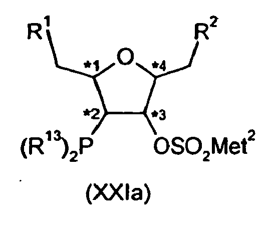 in der R¹, R² und R¹³ die unter der Formel (I) im Anspruch 1 und Met² die unter der Formel (XVII) im Anspruch 10 genannte Bedeutung besitzen.

18. Verbindungen der Formel (XXIb), 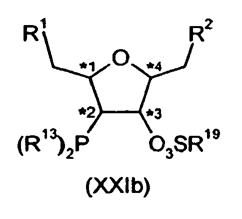 in der R¹, R² und R¹³ die unter der Formel (I) im Anspruch 1 genannte Bedeutung besitzen und R¹⁹ für C₁-C₁₂-Alkyl, C₁-C₁₂-Fluoralkyl, C₅-C₂₅₋Arylalkyl oder C₄-C₂₄-Aryl steht.

19. Übergangsmetallkomplexe enthaltend Verbindungen gemäß mindestens einem der Ansprüche 1 bis 10.

20. Übergangsmetallkomplexe gemäß Anspruch 19, **dadurch gekennzeichnet, dass** das Übergangsmetall ausgewählt ist aus der Gruppe Ruthenium, Osmium, Cobalt, Rhodium, Iridium, Nickel, Palladium, Platin und Kupfer.

21. Übergangsmetallkomplexe gemäß mindestens einem der Ansprüche 19 bis 20, **dadurch gekennzeichnet, dass** das molare Verhältnis von Übergangsmetall zu Verbindungen gemäß mindestens einem der Ansprüche 1 bis 10 1:1 beträgt.

22. Übergangsmetallkomplexe gemäß Anspruch 20, **dadurch gekennzeichnet dass** sie der der Formel (XXIII) gehorchen,
[(I)L¹₂M] (XXIII)
in der (I) für Verbindungen der Formel (I) mit der in Anspruch 1 genannten Bedeutung steht und
M für Rhodium oder Iridium und
L¹ jeweils für ein C₂-C₁₂-Alken oder ein Nitril oder
L¹₂ zusammen für ein (C₄-C₁₂)-Dien steht.

23. [Rh(cod)(2,3-bis-*O*-(Diphenylphosphino)-1,6-di-*O*-(triphenylmethyl)-2,5-anhydro-D-mannit)]BF₄, [Rh(cod)(2,3-bis-*O*-(Diphenylphosphino)-1,6-di-*O-*(*tert*-butyldiphenylsilyl)-2,5-anhydro-D-mannit)]BF₄, [Rh(cod)(2,3-bis-*O-*(Diphenylphosphino)-1,6-dideoxy-2,5-anhydro-D-mannit)]BF₄ und [Ir(cod)-(2,3-bis-*O*-(Diphenylphosphino)-1,6-di-*O*-(*tert*-butyldiphenylsilyl)-2,5-anhydro-D-mannit)]BF₄.

24. Übergangsmetallkomplexe gemäß mindestens einem der Ansprüche 19 bis 21, **dadurch gekennzeichnet, dass** sie durch Umsetzung von Übergangsmetallverbindungen und Verbindungen gemäß einem oder mehreren der Ansprüche 1 bis 10 gewonnen werden.

25. Übergangsmetallkomplexe gemäß Anspruch 24, **dadurch gekennzeichnet, dass** als Übergangsmetallverbindungen eingesetzt werden:
Übergangsmetallverbindungen der Formel (XXIIa)
M(An¹)_{q} (XXIIa)
in der
M für Rhodium, Iridium, Ruthenium, Nickel, Paladium, Platin oder Kupfer und
An¹ für Chlorid, Bromid, Acetat, Nitrat, Methansulfonat, Trifluormethansulfonat oder Acetylacetonat und
q für Rhodium, Iridium und Ruthenium für 3, für Nickel, Palladium und Platin für 2 und für Kupfer für 1 steht,
oder Übergangsmetallverbindungen der Formel (XXIIb),
M(An²)_{q}L¹₂ (XXIIb)
in der
M für Ruthenium, Iridium, Ruthenium, Nickel, Paladium, Platin oder Kupfer und
An² für Chlorid, Bromid, Acetat, Methansulfonat oder Trifluormethansulfonat, Tetrafluoroborat oder Hexafluorophosphat, Perchlorat, Hexafluoroantimonat, Tetra(bis-3,5-trifluromethylphenyl)-borat oder Tetraphenylborat steht und
q für Rhodium und Iridium für 1, für Ruthenium, Nickel, Palladium und Platin für 2 und für Kupfer für 1 steht,
L¹ jeweils für ein C₂-C₁₂-Alken steht oder
L¹₂ zusammen für ein (C₄-C₁₂)-Dien steht
oder Übergangsmetallverbindungen der Formel (XXIIc)
[ML²An¹₂]₂ (XXIIc)
in der
M für Ruthenium und
L² für Arylreste oder Cyclooctadien, Norbomadien oder Methylallyl steht
oder Übergangsmetallverbindungen der Formel (XXIId),
Met³_{q}[M(An³)₄] (XXIId)
in der
M für Palladium, Nickel, iridium oder Rhodium und
An³ für Chlorid oder Bromid steht und
Met³ für Lithium, Natrium, Kalium, Ammonium oder organisches Ammonium steht und
q für Rhodium und Iridium für 3, für Nickel, Palladium und Platin für 2 steht,
oder Übergangsmetallverbindungen der Formel (XXIIe),
[M(L³)₂]An⁴ (XXIIe)
in der
M für Iridium oder Rhodium und
L³ für (C₄-C₁₂)-Dien steht und
An⁴ für ein nicht oder schwach koordinierendes Anion steht
oder
Ni(1,5-Cyclooctadien)₂, Pd₂(dibenzylidenaceton)₃, Pd[PPh₃]₄, Cyclopentadienyl₂Ru, Rh(acac)(CO)₂, Ir(pyridin)2(1,5-Cyclooctadien), Cu(Phenyl)Br, Cu(Phenyl)Cl, Cu(Phenyl)I, Cu(PPh3)₂Br, [Cu(CH₃CN)₄]BF₄ und [Cu(CH₃CN)₄]PF₆ oder mehrkernige verbrückte Komplexe wie beispielsweise [Rh(1,5-cyclooctadien)Cl]₂, [Rh(1,5-cyclooctadien)Br]₂, [Rh(Ethen)₂Cl]₂ oder [Rh(Cycloocten)₂Cl]₂.

26. Übergangsmetallkomplexe gemäß Anspruch 25, **dadurch gekennzeichnet, dass** als Übergangsmetallverbindungen eingesetzt werden: [Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bistrifluormethylphenyl), [Rh(cod)₂]SbF₆, RuCl₂(cod), [(Cymol)RuCl₂]₂, [(Benzol)RuCl₂]₂, [(Mesityl)RuCl₂]₂, [(Cymol)RuBr₂]₂, [(Cymol)RuI₂]₂, [(Cymol)Ru(BF₄)₂]₂, [(Cymol)Ru(PF₆)₂]₂, [(Cymol)Ru(BAr₄)₂]₂ (Ar = 3,5-bistrifluormethylphenyl), [(Cymol)Ru(SbF₆)₂]₂, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bistrifluormethylphenyl), RuCl₃, NiCl₃, RhCl₃, PdCl₂, PdBr₂, Pd(OAc)2, Pd₂(dibenzylidenaceton)₃, Pd(acetylacetonat)₂, CuOTf, CuI, CuCl, Cu(OTf)₂, CuBr, CuI, CuBr₂, CuCl₂, CuI₂, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluormethylphenyl), [Rh(nbd)₂]SbF₆, RuCl₂(nbd), [Ir(nbd)₂]PF₆, [Ir(nbd)₂]ClO₄, [Ir(nbd)₂]SbF₆, [Ir(nbd)₂]BF₄, [Ir(nbd)₂]OTf, [Ir(nbd)₂]BAr₄ (Ar = 3,5-bistrifluormethylphenyl), Ir(pyridin)₂(nbd), [Ru(DMSO)₄Cl₂], [Ru(CH₃CN)₄Cl₂], [Ru(PhCN)₄Cl₂], [Ru(cod)Cl₂]ₙ, [Ru(cod)₄(Methallyl)₂], [Ru(acetylacetonat)₃].

27. Übergangsmetallkomplexe gemäß Anspruch 26, **dadurch gekennzeichnet, dass** als Übergangsmetallverbindungen eingesetzt werden:
[Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bistrifluormethylphenyl), [Rh(cod)₂]SbF₆, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluormethylphenyl), [Rh(nbd)₂]SbF₆, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bistrifluormethylphenyl).

28. Übergangsmetallkomplexe gemäß mindestens einem der Ansprüche 23 bis 27, **dadurch gekennzeichnet, dass** die Menge der eingesetzten Übergangsmetallverbindungen 25 bis 200 mol-% bezogen auf die eingesetzte Verbindung gemäß einem oder mehreren der Ansprüche 1 bis 10 beträgt.

29. Verwendung von Übergangsmetallkomplexen gemäß einem oder mehreren der Ansprüche 19 bis 28 zur Herstellung von stereoisomerenangereicherten Verbindungen.

30. Verwendung gemäß Anspruch 29, **dadurch gekennzeichnet, dass** die stereoisomerenangereicherte Verbindungen durch asymmetrische 1,4-Additionen, asymmetrische Hydroformylierungen, asymmetrische Hydrocyanierungen, asymmetrische Heck-Reaktionen und asymmetrische Hydrogenierungen erhalten werden.

31. Verwendung gemäß einem oder mehreren der Ansprüche 29 und 30, **dadurch gekennzeichnet, dass** die stereoisomerenangereicherten Verbindungen zur Herstellung von Wirkstoffen in Arzneimitteln und Agrochemikalien, oder Zwischenprodukten dieser beiden Klassen verwendet werden.

32. Verwendung von Übergangsmetallkomplexen gemäß einem oder mehreren der Ansprüche 19 bis 28 als Katalysatoren.

33. Verfahren zur Herstellung von stereoisomerenangereicherten Verbindungen durch katalytische Hydrierungen von Olefinen, Enaminen, Enamiden, Iminen oder Ketonen, 1,4-Additionen, Hydroformylierungen, Hydrocyanierungen oder Heck-Reaktionen, **dadurch gekennzeichnet, dass** als Katalysatoren solche verwendet werden, die Übergangsmetallkomplexe gemäß einem oder mehreren der Ansprüche 19 bis 28 enthalten.

34. Verfahren gemäß Anspruch 33, **dadurch gekennzeichnet, dass** die Menge der eingesetzten Übergangsmetallkomplexe 0.001 bis 5 mol-% bezogen auf das eingesetzte Substrat beträgt.

35. Verfahren gemäß einem oder mehreren der Ansprüche 33 bis 35, **dadurch gekennzeichnet, dass** die stereoisomerenangereicherten Verbindungen durch katalytische Hydrierung von Olefinen, Enamiden oder Iminen gewonnen werden.

36. Verfahren gemäß einem oder mehreren der Ansprüche 33 bis 35, **dadurch gekennzeichnet, dass** bei einer Temperatur von -20°C bis 200°C gearbeitet wird.

37. Verfahren gemäß einem oder mehreren der Ansprüche 34 bis 36, **dadurch gekennzeichnet, dass** der Wasserstoffdruck 0,1 bis 200 bar beträgt.

38. Katalysatoren enthaltend Übergangsmetallkomplexe gemäß einem oder mehreren der Ansprüche 19 bis 28.

## Claims

1. Compounds of the formula (I), 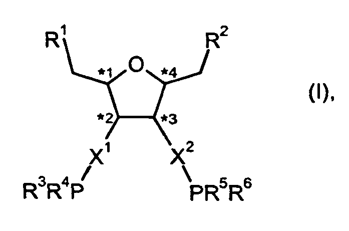 where
• *1, *2, *3 and *4 are each independently a stereogenic carbon atom which is in the R- or S- configuration,
• X¹ and X² are each independently absent or are oxygen and
• R¹ and R² may each independently be: hydrogen, C₁-C₂₀-alkyl, C₁-C₂₀₋fluoroalkyl, C₂-C₂₀-alkenyl, C₄-C₂₄-aryl, C₅-C₂₅-arylalkyl, C₆-C₂₆₋arylalkenyl or NR⁷R⁸, OR⁸, -(C₁-C₈-alkyl)-OR⁸, -(C₁-C₈-alkyl)-NR⁷R⁸ or -O₂CR⁸ where R⁷ and R⁸ are each independently C₁-C₈-alkyl, C₅₋C₁₄-arylalkyl or C₄-C₁₅-aryl, or R⁷ and R⁸ together are a cyclic amino radical having a total of 4 to 20 carbon atoms,
or R¹ and R² are each independently radicals of the formula (II)
-R⁹-SiR¹⁰R¹¹R¹² (II)
where
R⁹ is absent, or is oxygen or methylene and
R¹⁰, R¹¹ and R¹² are each independently C₁-C₁₂-alkyl, C₅-C₁₅-arylalkyl or C₄-C₁₄-aryl and
• R³, R⁴, R⁵ and R⁶ are each independently R¹³, OR¹⁴ or NR¹⁵R¹⁶ where R¹³, R¹⁴, R¹⁵ and R¹⁶ are each independently C₁-C₁₂-alkyl, C₅-C₁₅₋arylalkyl or C₄-C₁₄-aryl, or NR¹⁵R¹⁶ together is a cyclic amino radical having 4 to 20 carbon atoms, or R³ and R⁴ and/or R⁵ and R⁶ in each case together are -O-R¹⁷-O- where R¹⁷ is a radical selected from the group of C₂-C₄-alkylene, 1,2-phenylene, 1,3-phenylene, 1,2-cyclohexylene, 1,1'-ferrocenylene, 1,2-ferrocenylene, 2,2'-(1,1'-binaphthylene), 2,2'-(1,1')-biphenylene and 1,1'-(diphenyl-2,2'-methylene)-diyl, and the radicals mentioned may optionally be mono- or polysubstituted by radicals selected from the group of fluorine, chlorine, C₁-C₈-alkoxy and C₁-C₈-alkyl.

2. Compounds according to Claim 1, where, in formula (I), ^{*1},^{*2},^{*3},^{*4} together define the following stereoisomers of the central substituted furan ring:
(1R,2R,3R,4R), (1R,2R,3R,4S), (1R,2R,3S,4S), (1R,2S,3S,4S), (1R,2S,3R,4S), (1R,2S,3S,4R), (1R,2R,3S,4R), (1S,2S,3R,4S), (1S,2S,3S,4S), (1S,2S,3S,4R), (1S,2S,3R,4R), (1S,2R,3R,4R), (1S,2R,3S,4R), (1S,2R,3R,4S), (1S,2S,3R,4S), (1R,2R,3S,4R).

3. Compounds according to at least one of Claims 1 to 2, **characterized in that**, in formula (I), R¹ and R² are each independently hydrogen, C₁-C₄-alkyl, C₄₋C₁₄-aryl, O-R⁸, O₂C-R⁸ where R⁸ is preferably C₁-C₁₂-alkyl, C₅-C₂₅-arylalkyl or C₄-C₁₄-aryl, or OSiR¹⁰R¹¹R¹², and R¹⁰, R¹¹, and R¹² are preferably each independently C₁-C₁₂-alkyl or C₄-C₁₄-aryl.

4. Compounds according to at least one of Claims 1 to 3, **characterized in that**, in formula (I), R¹ and R² are each independently hydrogen, tert-butoxy, trityloxy, tert-butyldimethylsilyloxy, tert-butyldiphenylsilyloxy, trimethylsilyloxy, triethylsilyloxy, triisopropylsilyloxy, neopentoxy or 1-adamantoxy.

5. Compounds according to at least one of Claims 1 to 4, **characterized in that**, in formula (I), R³, R⁴, R⁵ and R⁶ are each independently R¹³, OR¹⁴ or NR¹⁵R¹⁶ where R¹³, R¹⁴, R¹⁵ and R¹⁶ are each independently C₁-C₁₂-alkyl or C₄-C₁₄₋aryl, or NR¹⁵R¹⁶ together is a cyclic amino radical having 4 to 12 carbon atoms, or R³ and R⁴ and/or R⁵ and R⁶ together are each -O-R¹⁷-O- where R¹⁷ is ethylene, 1,2-phenylene, 1,3-phenylene, 1,2-cyclohexylene, 1,1'-ferrocenylene, 1,2-ferrocenylene, di- or tetra- C₁-C₈-alkyl-substituted 1,1'-(diphenyl-2,2'-methylene)-diyl, 2,2'-(1,1'-binaphthylene) or 2,2'-(1,1')-biphenylene, and 2,2'-(1,1'-binaphthylene) or 2,2'-(1,1')-biphenylene is substituted at least in the 6,6'-position by radicals selected from the group of C₁-C₈-alkoxy and C₁-C₈-alkyl, and may also be substituted in the 5,5'-,4,4'-, 3,3'- or 2,2'-position by radicals selected from the group of fluorine, chlorine, C₁-C₈-alkoxy and C₁-C₈-alkyl.

6. Compounds according to at least one of Claims 1 to 5, **characterized in that**, in formula (I), R³, R⁴, R⁵ and R⁶ are each independently R¹³, OR¹⁴ or NR¹⁵R¹⁶, where R¹³ and R¹⁴ are each independently methyl, ethyl, n-propyl, isopropyl, tert-butyl, cyclohexyl, phenyl, 2-(C₁-C₈)-alkylphenyl, 3-(C₁-C₈)-alkylphenyl, 4-(C₁-C₈)-alkylphenyl, 2,6-di-(C₁-C₈)-alkylphenyl, 3,5-di-(C₁₋C₈)-alkylphenyl, 2,4-di-(C₁-C₈)-alkylphenyl, 3,4,5-tri-(C₁-C₈)-alkylphenyl, 2-(C₁-C₈)-alkoxyphenyl, 3-(C₁-C₈)-alkoxyphenyl, 4-(C₁-C₈)-alkoxyphenyl, 2,4-di-(C₁-C₈)-alkoxyphenyl, 2,6-di-(C₁-C₈)-alkoxyphenyl, 3,5-di-(C₁-C₈)-alkoxyphenyl, 3,4,5-tri-(C₁-C₈)-alkoxyphenyl, 3,5-dialkyl-4-(C₁-C₈)-alkoxyphenyl, 3,5-(C₁-C₈)-dialkyl-4-di-(C₁-C₈)-alkylaminophenyl, 4-di-(C₁₋C₈)-alkylaminophenyl, 3,5-bis-((C₁-C₄)-fluoroalkyl), 2,4-bis-((C₁-C₄)-fluoroalkyl)phenyl, 4-((C₁-C₄)-fluoroalkyl)phenyl and mono-, di-, tri- or tetrafluorine- and/or -chlorine-substituted phenyl, fluorenyl or naphthyl or NR¹⁵R¹⁶ as a whole is dimethylamino, diethylamino, pyrrolidino or diisopropylamino or R³ and R⁴ and/or R⁵ and R⁶, each in pairs, are O-R¹⁷-O where R¹⁷ is 1,1'-bis-(4,6-di-(C₁-C₈-alkyl)-phenyl)-2,2'-methylene)-diyl or where R¹⁷ is (R)-1,1'-biphenyl-2,2'-diyl, (S)-1,1'-biphenyl-2,2'-diyl, (R)-1,1'-binaphthyl-2,2'-diyl, (S)-1,1'-binaphthyl-2,2'-diyl, 1,1'-[bis-(4-methyl-6-tert-butylphenyl)-2,2'-methylene)]-diyl or 1,1'-[bis-(4-methyl-6-(1-methylcyclohexyl)-2,2'-methylene)]-diyl.

7. Compounds according to at least one of Claims 1 to 6, **characterized in that**, in formula (I), R³ and R⁴ and/or R⁵ and R⁶ in pairs are identical.

8. Compounds according to at least one of Claims 1 to 2, **characterized in that** they obey the formula (Ia) to (Ii) 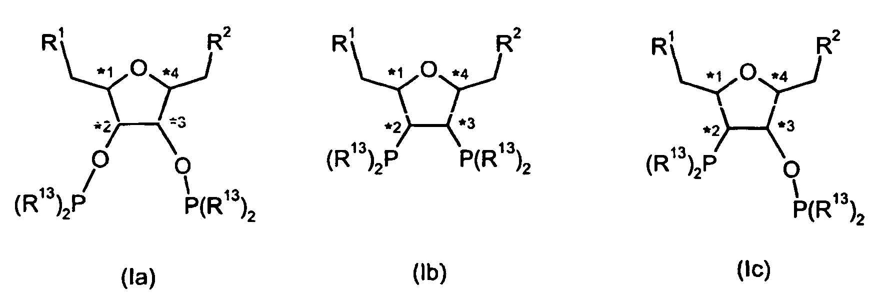 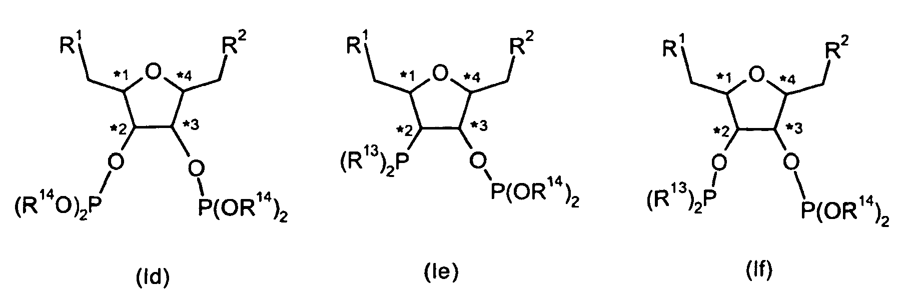 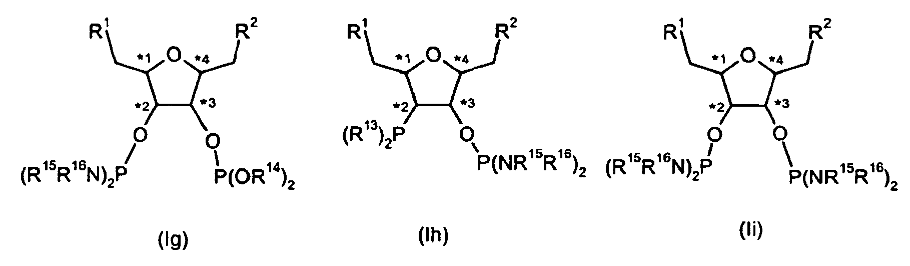 where *¹,*²,*³,*⁴, R¹, R², R¹³, R¹⁴, R¹⁵ and R¹⁶ are each as defined under formula (I) in Claim 1.

9. Compounds of the formula (XIII), 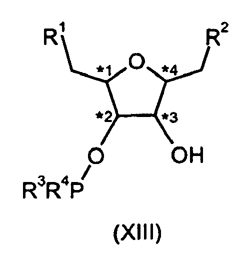 where R¹, R², R³ and R⁴ are each as defined under formula (I) in Claim 1.

10. 2,3-bis-*O*-(Diphenylphosphino)-1,6-di-*O*-(triphenylmethyl)-2,5-anhydro-D-mannitol, 2,3-bis-*O*-(diphenylphosphino)-1,6-dideoxy-2,5-anhydro-D-mannitol, 2,3-bis-*O*-(diphenylphosphino)-1,6-di-*O*-(*tert*-butyldiphenylsilyl)-2,5-anhydro-D-mannitol, 2,3-bis-*O*-(diphenylphosphino)-1,6-di-*O-*(triphenylmethyl)-2,5-anhydro-L-iditol, 2,3-bis-*O*-(diphenylphosphino)-1,6-di-*O*-(*tert*-butyldiphenylsilyl)-2,5-anhydro-L-iditol, 2,3-bis-*O-*(diphenylphosphino)-1,6-dideoxy-2,5-anhydro-L-iditol, 2,3-bis-*O*-(di(4-methoxyphenyl)phosphino)-1,6-di-*O*-(*tert*-butyldiphenylsilyl)-2,5-anhydro-D-mannitol, 2,3-bis-*O*-(di((4-Trifluoromethyl)phenyl)phosphino)-1,6-di-*O*-(*tert-*butyldiphenylsilyl)-2,5-anhydro-D-mannitol, 2-*O*-(di(2,4-dimethylphenyl)phosphino)-3-*O*-(diphenylphosphino)-1,6-di-*O*-(*tert-*butyldiphenylsilyl)-2,5-anhydro-D-mannitol, 2-*O*-(di(2,4-dimethylphenyl)phosphino)-3-*O*-(4,8-ditert-butyl-2,10-dimethyl-12H-dibenzo[δ,γ][1,3,2]dioxaphosphocino)-1,6-di-*O*-(*tert*-butyldiphenylsilyl)-2,5-anhydro-D-mannitol and 2-*O*-(di(2,4-dimethylphenyl)phosphino)-3-*O*-(2,10-dimethyl-4,8-bis(1-methylcyclohexyl)-12H-dibenzo[δ,γ]-[1,3,2]dioxaphosphocino)-1,6-di-*O*-(*tert*-butyldiphenylsilyl)-2,5-anhydro-D-mannitol.

11. Process for preparing compounds of the formula (XV) 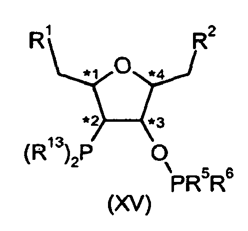 where R¹, R²,R⁵, R⁶ and R¹³ are each as defined under formula (I) in Claim 1,
**characterized in that**,
in step a)
compounds of the formula (XVI) 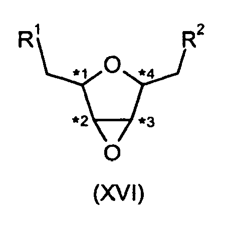 where R¹ and R² are each defined under formula (I) in Claim 1, in the presence of compounds of the formula (XVII)
(R¹³)₂PMet² (XVII)
where
Met² is lithium, sodium or potassium and
R¹³ is as defined under formula (I) in Claim 1,
are converted to compounds of the formula (XVIII) 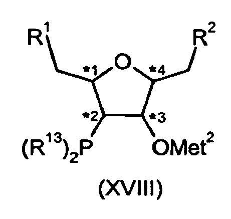 where R¹, R², Met² and R¹³ are as defined above,
and, in step b),
the compounds of the formula (XVIII) are reacted with compounds of the formula (XIIb)
R⁵R⁶P-Y (XIIb)
where R⁵ and R⁶ are each as defined under formula (I) in Claim 1 and
Y is chlorine, bromine, iodine, dimethylamino or diethylamino, to give compounds of the formula (XV).

12. Process according to Claim 11, **characterized in that** the compounds of the formula (XVII) are converted by acidifying to compounds of the formula (XIX) 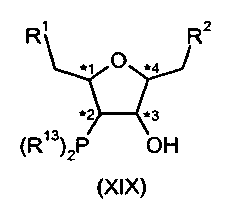 and, in step b), are converted by reacting with compounds of the formula (XIIb) to compounds of the formula (XV).

13. Process according to Claim 12, **characterized in that** step b) is carried out in the presence of a base.

14. Compounds of the formula (XVIII) 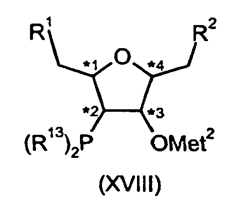 where R¹, R² and R¹³ are each as defined under formula (I) in Claim 1 and Met² is as defined under formula (XVII) in Claim 10.

15. Compounds of the formula (XIX) 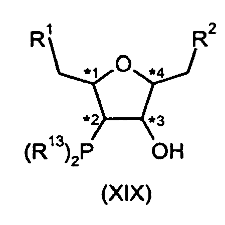 where R¹, R² and R¹³are each as defined under formula (I) in Claim 1.

16. Compounds of the formula (XXa) 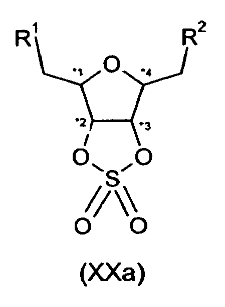 where R¹ and R² are each as defined under formula (I) in Claim 1.

17. Compounds of the formula (XXIa), 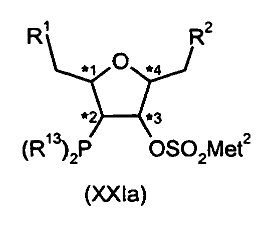 where R¹, R² and R¹³ are each as defined under formula (I) in Claim 1 and Met² is as defined under formula (XVII) in Claim 10.

18. Compounds of the formula (XXIb), 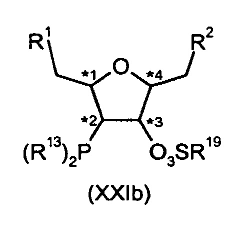 where R¹, R² and R¹³ are each as defined under formula (I), and R¹⁹ is C₁-C₁₂₋alkyl, C₁-C₁₂-fluoroalkyl, C₅-C₂₅-arylalkyl or C₄-C₂₄-aryl.

19. Transition metal complexes containing compounds according to at least one of Claims 1 to 10.

20. Transition metal complexes according to Claim 19, **characterized in that** the transition metal is selected from the group of ruthenium, osmium, cobalt, rhodium, iridium, nickel, palladium, platinum and copper.

21. Transition metal complexes according to at least one of Claims 19 to 20, **characterized in that** the molar ratio of transition metal to compounds according to at least one of Claims 1 to 10 is 1:1.

22. Transition metal complexes according to Claim 20, **characterized in that** they obey the the formula (XXIII)
[(I)L¹₂M] (XXIII)
where (I) represents a compound of the formula (I) as defined in Claim 1 and
M is rhodium or iridium and
L¹ is in each case a C₂-C₁₂-alkene or a nitrile or
L¹₂ together is a (C₄-C₁₂)-diene.

23. [Rh(cod)(2,3-bis-*O*-(diphenylphosphino)-1,6-di-*O*-(triphenylmethyl)-2,5-anhydro-D-mannitol)]BF₄, [Rh(cod)(2,3-bis-*O*-(diphenylphosphino)-1,6-di-*O*-(*tert*-butyldiphenylsilyl)-2,5-anhydro-D-mannitol)]BF₄, [Rh(cod)(2,3-bis-*O*-(diphenylphosphino)-1,6-dideoxy-2,5-anhydro-D-mannitol)]BF₄ and [Ir(cod)(2,3-bis-*O*-(diphenylphosphino)-1,6-di-*O*-(*tert*-butyldiphenylsilyl)-2,5-anhydro-D-mannitol)]BF₄.

24. Transition metal complexes according to at least one of Claims 19 to 21, **characterized in that** they are obtained by reacting transition metal compounds and compounds according to at least one of Claims 1 to 10.

25. Transition metal complexes according to Claim 24, **characterized in that** the transition metal compounds used are:
transition metal compounds of the formula (XXIIa)
M(An¹)_{q} (XXIIa)
where
M is rhodium, iridium, ruthenium, nickel, palladium, platinum or copper and
An¹ is chloride, bromide, acetate, nitrate, methanesulphonate, trifluoromethanesulphonate or acetylacetonate and
q is 3 for rhodium, iridium and ruthenium, is 2 for nickel, palladium and platinum, and is 1 for copper,
or transition metal compounds of the formula (XXIIb),
M(An²)_{q}L¹₂ (XXIIb)
where
M is rhodium, iridium, ruthenium, nickel, paladium, platinum or copper and
An² is chloride, bromide, acetate, methanesulphonate or trifluoromethanesulphonate, tetrafluoroborate or hexafluorophosphate, perchlorate, hexafluoroantimonate, tetra(bis-3,5-trifluromethylphenyl)borate or tetraphenylborate and
q is 1 for rhodium and iridium, is 2 for ruthenium, nickel, palladium and platinum, and is 1 for copper,
L¹ is in each case a C₂-C₁₂-alkene or
L¹₂ together is a (C₄-C₁₂)-diene
or transition metal compounds of the formula (XXIIc)
[ML²An¹₂]₂ (XXIIc)
where
M is ruthenium and
L² is an aryl radical or cyclooctadiene, norbomadiene or methylallyl
or transition metal compounds of the formula (XXIId)
Met³_{q}[M(An³)₄] (XXIId)
where
M is palladium, nickel, iridium or rhodium and
An³ is chloride or bromide and
Met³ is lithium, sodium, potassium, ammonium or organic ammonium and
q is 3 for rhodium and iridium, and is 2 for nickel, palladium and platinum,
or transition metal compounds of the formula (XXIIe)
[M(L³)₂]An⁴ x (XXIId)
where
M is iridium or rhodium and
L³ is (C₄-C₁₂)-diene and
An⁴ is a noncoordinating or weakly coordinating anion
or
Ni(1,5-cyclooctadiene)₂, Pd₂(dibenzylideneacetone)₃, Pd[PPh₃]₄, cyclopentadienyl₂Ru, Rh(acac)(CO)₂, Ir(pyridine)2(1,5-cyclooctadiene), Cu(phenyl)Br, Cu(phenyl)Cl, Cu(phenyl)I, Cu(PPh3)₂Br, [Cu(CH₃CN)₄]BF₄ and [Cu(CH₃CN)₄]PF₆ or multinuclear bridged complexes, for example [Rh(1,5-cyclooctadiene)Cl]₂, [Rh(1,5-cyclooctadiene)Br]₂, [Rh(ethene)₂Cl]₂ or [Rh(cyclooctene)₂Cl]₂.

26. Transition metal complexes according to Claim 25, **characterized in that** the transition metal compounds used are: [Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bistrifluoromethylphenyl), [Rh(cod)₂]SbF₆, RuCl₂(cod), [(cymene)RuCl₂]₂, [(benzene)RuCl₂]₂, [(mesityl)RuCl₂]₂, [(cymene)RuBr₂]₂, [(cymene)RuI₂]₂, [(cymene)Ru(BF₄)₂]₂, [(cymene)Ru(PF₆)₂]₂, [(cymene)Ru(BAr₄)₂]₂ (Ar = 3,5-bistrifluoromethylphenyl), [(cymene)Ru(SbF₆)₂]₂, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bistrifluoromethylphenyl), RuCl₃, NiCl₃, RhCl₃, PdCl₂, PdBr₂, Pd(OAc)2, Pd₂(dibenzylideneacetone)₃, Pd(acetylacetonate)₂, CuOTf, CuI, CuCl, Cu(OTf)₂, CuBr, CuI, CuBr₂, CuCl₂, CuI₂, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluoromethylphenyl), [Rh(nbd)₂]SbF₆, RuCl₂(nbd), [Ir(nbd)₂]PF₆, [Ir(nbd)₂]ClO₄, [Ir(nbd)₂]SbF₆, [Ir(nbd)₂]BF₄, [Ir(nbd)₂]OTf, [Ir(nbd)₂]BAr₄ (Ar = 3,5-bistrifluoromethylphenyl), Ir(pyridine)₂(nbd), [Ru(DMSO)₄Cl₂], [Ru(CH₃CN)₄Cl₂], [Ru(PhCN)₄Cl₂], [Ru(cod)Cl₂]ₙ, [Ru(cod)₄(methallyl)₂], [Ru(acetylacetonate)₃].

27. Transition metal complexes according to Claim 26, **characterized in that** the transition metal compounds used are:
[Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bistrifluoromethylphenyl), [Rh(cod)₂]SbF₆, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluoromethylphenyl), [Rh(nbd)₂]SbF₆, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bistrifluoromethylphenyl).

28. Transition metal complexes according to at least one of Claims 23 to 27, **characterized in that** the amount of the transition metal compounds used is 25 to 200 mol%, based on the compound according to one or more of Claims 1 to 10 used.

29. Use of transition metal complexes according to one or more of Claims 19 to 28 for preparing stereoisomerically enriched compounds.

30. Use according to Claim 29, **characterized in that** the stereoisomerically enriched compounds are obtained by asymmetric 1,4-additions, asymmetric hydroformylations, asymmetric hydrocyanations, asymmetric Heck reactions and asymmetric hydrogenations.

31. Use according to one or more of Claims 29 and 30, **characterized in that** the stereoisomerically enriched compounds are used for preparing active ingredients in pharmaceuticals and agrochemicals, or intermediates of these two classes.

32. Use of transition metal complexes according to one or more of Claims 19 to 28 as catalysts.

33. Process for preparing stereoisomerically enriched compounds by catalytic hydrogenations of olefins, enamines, enamides, imines or ketones, 1,4-additions, hydroformylations, hydrocyanations or Heck reactions, **characterized in that** the catalysts used are those which comprise transition metal complexes according to one or more of Claims 19 to 28.

34. Process according to Claim 33, **characterized in that** the amount of the transition metal complexes used is 0.001 to 5 mol%, based on the substrate used.

35. Process according to one or more of Claims 33 and 35, **characterized in that** the stereoisomerically enriched compounds are obtained by catalytic hydrogenation of olefins, enamides or imines.

36. Process according to one or more of Claims 33 to 35, **characterized in that** the working temperature is -20°C to 200°C.

37. Process according to one or more of Claims 34 to 36, **characterized in that** the hydrogen pressure is 0.1 to 200 bar.

38. Catalysts comprising transition metal complexes according to one or more of Claims 19 to 28.

## Revendications

1. Composé de formule (I) 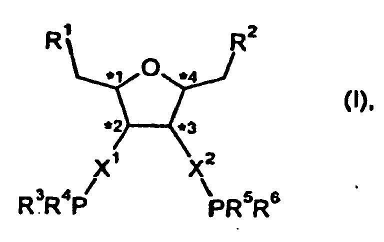 dans laquelle
• ^{*1}, *^{2, *3} et ^{*4} désignent chacun, indépendamment les uns des autres, un atome de carbone stéréogène qui se trouve en configuration R ou S,
• X¹ et X², chacun indépendamment l'un de l'autre, sont absents ou représentent un atome d'oxygène et
• R¹ et R² peuvent représenter chacun, indépendamment l'un de l'autre : un atome d'hydrogène, un groupe alkyle en C₁-C₂₀, fluoroalkyle en C₁-C₂₀, alcényle en C₂-C₂₀, aryle en C₄-C₂₄, arylalkyle en C₅-C₂₅, arylalcényle en C₆-C₂₆ ou NR⁷R⁸, OR⁸, - [alkyl(C₁-C₈)]-OR⁸, -[alkyl(C₁-C₈)]-NR⁷R⁸ ou -O₂CR⁸, R⁷ et R⁸ représentant chacun, indépendamment l'un de l'autre, un groupe alkyle en C₁-C₈, arylalkyle en C₅-C₁₄ ou aryle en C₄-C₁₅, ou R⁷ et R⁸ forment ensemble un radical amino cyclique ayant au total de 4 à 20 atomes de carbone,
ou R¹ et R², chacun indépendamment l'un de l'autre, représentent des radicaux de formule (II)
-R⁹-SiR¹⁰R¹¹R¹² (II)
dans laquelle
R⁹ est absent ou représente un atome d'oxygène ou le groupe méthylène et
R¹⁰, R¹¹ et R¹² représentent chacun indépendamment un groupe alkyle en C₁-C₁₂, arylalkyle en C₅-C₁₅ ou aryle en C₄-C₁₄, et
• R³, R⁴, R⁵ et R⁶ peuvent représenter chacun, indépendamment les uns des autres, R¹³, OR¹⁴ ou NR¹⁵R¹⁶, R¹³, R¹⁴, R¹⁵ et R¹⁶ représentant chacun, indépendamment les uns des autres, un groupe alkyle en C₁-C₁₂, arylalkyle en C₅-C₁₅ ou aryle en C₄-C₁₄, ou NR¹⁵R¹⁶ représentent ensemble un radical amino cyclique ayant de 4 à 20 atomes de carbone, ou bien R³ et R⁴ ou, respectivement, R⁵ et R⁶ représentent respectivement ensemble -O-R¹⁷-O-, R¹⁷ représentant des radicaux qui sont choisis dans l'ensemble constitué par les groupes alkylène en C₂-C₄, 1,2-phénylène, 1,3-phénylène, 1,2-cyclohexylène, 1,1'-ferrocénylène, 1,2-ferrocénylène, 2,2'-(1,1'-binaphtylène), 2,2'-(1,1'-biphénylène) et 1,1'-(diphényl-2,2'-méthylène)diyle, les radicaux cités pouvant éventuellement être substitués une ou plusieurs fois par des radicaux qui sont choisis dans l'ensemble constitué par les atomes de fluor et de chlore et des groupes alcoxy en C₁-C₈ et alkyle en C₁-C₈.

2. Composés selon la revendication 1, dans lesquels, dans la formule (I), ^{*1}, ^{*2}, ^{*3}, ^{*4} définissent ensemble les stéréoisomères suivants du cycle furanne central substitué :
| | | |
|---|---|---|
| (1R, 2R, 3R, 4R), | (1R, 2R, 3R, 4S), | (1R, 2R, 3S, 4S), |
| (1R, 2S, 3S, 4S), | (1R, 2S, 3R, 4S), | (1R, 2S, 3S, 4R), |
| (1R, 2R, 3S, 4R), | (1S, 2S, 3R, 4S), | (1S, 2S, 3S, 4S), |
| (1S, 2S, 3S, 4R), | (1S, 2S, 3R, 4R), | (1S, 2R, 3R, 4R), |
| (1S, 2R, 3S, 4R), | (1S, 2R, 3R, 4S), | (1S, 2S, 3R, 4S), |
| (1R, 2R, 3S, 4R). | | |

3. Composés selon au moins l'une des revendications 1 et 2, **caractérisé en ce que**, dans la formule (I), R¹ et R² représentent chacun, indépendamment l'un de l'autre, un atome d'hydrogène ou un groupe alkyle en C₁-C₄, aryle en C₄-C₁₄, O-R⁸, O₂C-R⁸, R⁸ représentant de préférence un groupe alkyle en C₁-C₁₂, arylalkyle en C₅-C₂₅ ou aryle en C₄-C₁₄, ou OSiR¹⁰R¹¹R¹², R¹⁰, R¹¹ et R¹² de préférence représentant chacun indépendamment un groupe alkyle en C₁-C₁₂ ou aryle en C₄-C₁₄.

4. Composés selon au moins l'une des revendications 1 à 3, **caractérisés en ce que**, dans la formule (I), R¹ et R² représentent chacun indépendamment l'un de l'autre un atome d'hydrogène, le groupe tert-butoxy, trityloxy, tert-butyldiméthylsilyloxy, tert-butyldiphénylsilyloxy, triméthylsilyloxy, triéthylsilyloxy, triisopropylsilyloxy, néopentoxy ou 1-adamantoxy.

5. Composés selon au moins l'une des revendications 1 à 4, **caractérisés en ce que**, dans la formule (I), R³, R⁴, R⁵ et R⁶ représentent chacun, indépendamment les uns des autres, R¹³, OR¹⁴ ou NR¹⁵R¹⁶, R¹³, R¹⁴, R¹⁵ et R¹⁶ représentant chacun indépendamment un groupe alkyle en C₁-C₁₂ ou aryle en C₄-C₁₄, ou bien NR¹⁵R¹⁶ représentant ensemble un radical amino cyclique ayant de 4 à 12 atomes de carbone, ou bien R³ et R⁴ ou R⁵ et R⁶ représentent respectivement ensemble -O-R¹⁷-O-, R¹⁷ représentant un groupe éthylène, 1,2-phénylène, 1,3-phénylène, 1,2-cyclohexylène, 1,1'-ferrocénylène, 1,2-ferrocénylène, un groupe 1,1'-(diphényl-2,2'-méthylène)diyle substitué deux ou quatre fois par des groupes alkyle en C₁-C₈, 2 2'-(1,1'-binaphtylène) ou 2,2'-(1,1')-biphénylène, le groupe 2,2'-(1,1'-binaphtylène) ou 2,2'-(1,1')-biphénylène étant substitué au moins en position 6,6' par des radicaux qui sont choisis dans l'ensemble constitué par des groupes alcoxy en C₁-C₈ et alkyle en C₁-C₈ et en outre en position 5,5', 4,4', 3,3' ou 2,2' par des radicaux qui sont choisis dans l'ensemble constitué par les atomes de fluor et de chlore et des groupes alcoxy en C₁-C₈ et alkyle en C₁-C₈.

6. Composés selon au moins l'une des revendications 1 à 5, **caractérisés en ce que** dans la formule (I), R³, R⁴, R⁵ et R⁶ représentent chacun indépendamment les uns des autres R¹³, OR¹⁴ ou NR¹⁵R¹⁶, R¹³ et R¹⁴ représentant chacun indépendamment un groupe méthyle, éthyle, n-propyle, isopropyle, tert-butyle, cyclohexyle, phényle, 2-alkyl(C₁-C₈)phényle, 3-alkyl(C₁-C₈)phényle, 4-alkyl(C₁-C₈)phényle, 2,6-dialkyl(C₁-C₈)phényle, 3,5-dialkyl(C₁-C₈)phényle, 2,4-dialkyl(C₁-C₈)phényle, 3,4,5-trialkyl(C₁-C₈)-phényle, 2-alcoxy(C₁-C₈)phényle, 3-alcoxy(C₁-C₈)-phényle, 4-alcoxy(C₁-C₈)phényle, 2,4-dialcoxy-(C₁-C₈)phényle, 2,6-dialcoxy(C₁-C₈)phényle, 3,5-dialcoxy(C₁-C₈)phényle, 3,4,5-trialcoxy(C₁-C₈)-phényle, 3,5-dialkyl-4-alcoxy(C₁-C₈)phényle, 3,5-dialkyl(C₁-C₈)-4-dialkyl(C₁-C₈)aminophényle, 4-dialkyl(C₁-C₈)aminophényle, 3,5-bis(fluoroalkyle-(C₁-C₄)), 2,4-bis(fluoroalkyl(C₁-C₄))phényle, 4-(fluoroalkyl(C₁-C₉))phényle et un groupe phényle, fluorényle ou naphtyle une, deux, trois ou quatre fois substitué par des atomes de fluor et/ou de chlore, ou bien NR¹⁵R¹⁶ représentant ensemble le groupe diméthylamino, diéthylamino, pyrrolidino ou diisopropylamino, ou R³ et R⁴ ou, respectivement, R⁵ et R⁶ représentent respectivement en paires O-R¹⁷-O, R¹⁷ représentant le groupe 1,1'-bis(4,6-dialkyl-(C₁-C₈)phényl)-2,2'-méthylène-diyle, ou R¹⁷ représentant un groupe (R)-1,1'-biphényl-2,2'-diyle, (S)-1,1'-biphényl-2,2'-diyle, (R)-1,1'-binaphtyl-2,2'-diyle, (S)-1,1'-binaphtyl-2,2'-diyle, 1,1'-[bis(4-méthyl-6-tert-butylphényl)-2,2'-méthylène]-diyle ou 1,1'-[bis(4-méthyl-6-(1-méthylcyclohexyl)-2,2'-méthylène)]diyle.

7. Composés selon au moins l'une des revendications 1 à 6, **caractérisés en ce que**, dans la formule (I), R³ et R⁴ ou, respectivement, R⁵ et R⁶ sont identiques, en paires.

8. Composés selon au moins l'une des revendications 1 et 2, **caractérisés en ce qu'**ils correspondent aux formules (Ia) à (Ii) 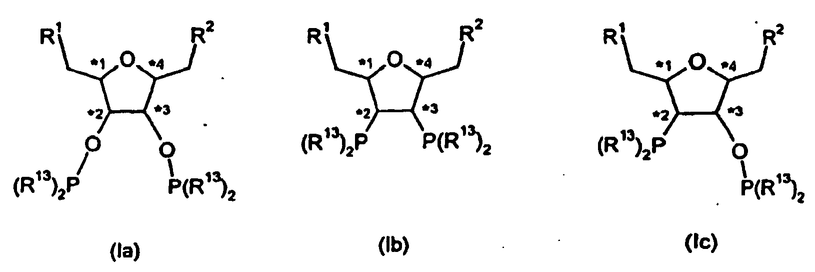 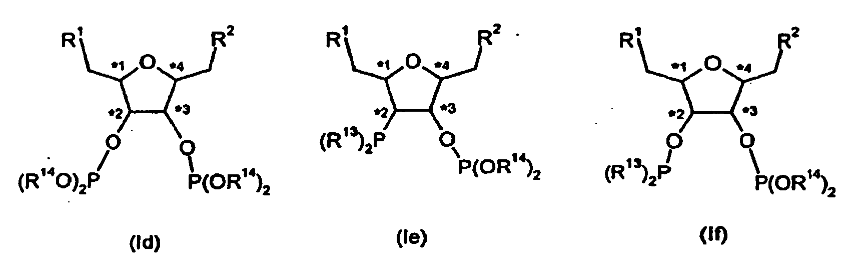 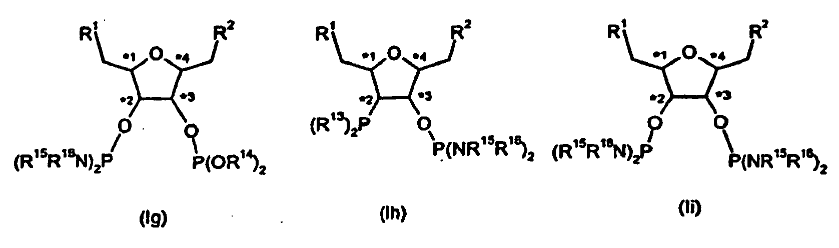 dans lesquelles *1, *2, *3, *4, R¹, R², R¹³, R¹⁴, R¹⁵ et R¹⁶ ont chacun la signification mentionnée sous la formule (I) dans la revendication 1.

9. Composés de formule (XIII), 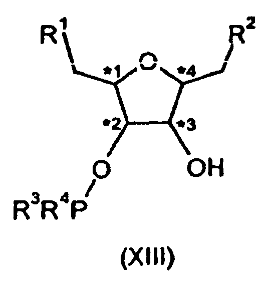 dans laquelle R¹, R², R³ et R⁴ ont les mêmes significations que celles indiquées sous la formule (I) dans la revendication 1.

10. 2,3-bis-O-(diphénylphosphino)-1,6-di-O-(triphénylméthyl)-2,5-anhydro-D-mannitol, 2,3-bis-O-(diphénylphosphino)-1,6-didésoxy-2,5-anhydro-D-mannitol, 2,3-bis-O-(diphénylphosphino)-1,6-di-O-(tert-butyldiphénylsilyl)-2,5-anhydro-D-mannitol, 2,3-bis-O-(diphénylphosphino)-1,6-di-O-(triphénylméthyl)-2,5-anhydro-L-iditol, 2,3-bis-O-(diphénylphosphino)-1,6-di-O-(tert-butyldiphénylsilyl)-2,5-anhydro-L-iditol, 2,3-bis-O-(diphénylphosphino)-1,6-didésoxy-2,5-anhydro-L-iditol, 2,3-bis-O-(di(4-méthoxyphényl)phosphino)-1,6-di-O-(tert-butyldiphénylsilyl)-2,5-anhydro-D-mannitol, 2,3-bis-O-(di((4-trifluorométhyl)phényl)-phosphino)-1,6-di-O-(tert-butyldiphénylsilyl)-2,5-anhydro-D-mannitol, 2-O-(di(2,4-diméthylphényl)-phosphino)-3-O-(diphénylphosphino)-1,6-di-O-(tert-butyldiphénylsilyl)-2,5-anhydro-D-mannitol, 2-0-(di(2,4-diméthylphényl)phosphino)-3-O-(4,8-di-tert-butyl-2,10-diméthyl-12H-dibenzo [δ,γ] [1,3,2] dioxaphosphocino)-1,6-di-O-(tert-butyldiphénylsilyl)-2,5-anhydro-D-mannitol et 2-O-(di(2,4-diméthylphényl)phosphino)-3-O-(2,10-diméthyl-4,8-bis(1-méthylcyclohexyl)-12H-dibenzo[δ,γ] [1,3,2] dioxaphosphocino)-1,6-di-O-(tert-butyldiphénylsilyl)-2,5-anhydro-D-mannitol.

11. Procédé pour la préparation de composés de formule (XV) 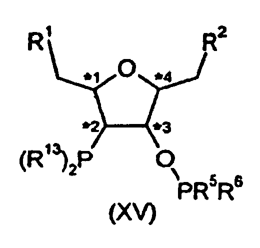 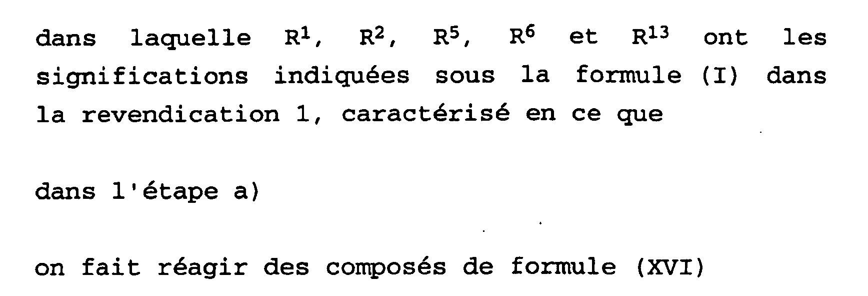 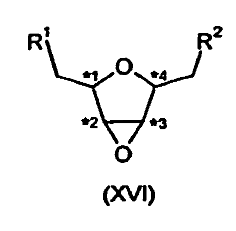 dans laquelle R¹ et R² ont les significations données sous la formule (I) dans la revendication 1,
en présence de composés de formule (XVII)
(R¹³)₂PMet² (XVII)
dans laquelle
Met² représente le lithium, le sodium ou le potassium et
R¹³ a la signification donnée sous la formule (I) dans la revendication 1,
pour obtenir des composés de formule (XVIII) 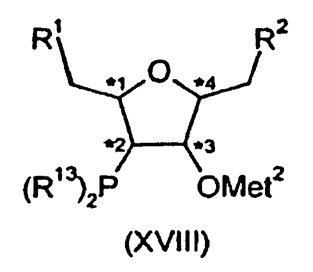 dans laquelle R¹, R², Met² et R¹³ ont les significations données précédemment,
et, dans l'étape b)
on fait réagir les composés de formule (XVIII) avec des composés de formule (XIIb)
R⁵R⁶P-Y (XIIb)
dans laquelle R⁵ et R⁶ ont les mêmes significations que celles indiquées sous la formule (I) dans la revendication 1 et
Y représente un atome de chlore, brome ou iode ou le groupe diméthylamino ou diéthylamino, pour obtenir des composés de formule (XV).

12. Procédé selon la revendication 11, **caractérisé en ce que** les composés de formule (XVII) sont convertis par acidification en composés de formule (XIX) 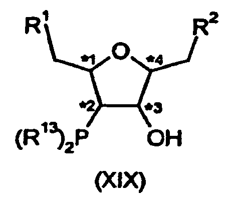 et sont convertis dans l'étape b), par réaction avec des composés de formule (XIIb), en composés de formule (XV).

13. Procédé selon la revendication 12, **caractérisé en ce que** l'étape b) est effectuée en présence d'une base.

14. Composés de formule (XVIII) 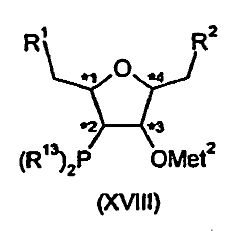 dans laquelle R¹, R² et R¹³ ont les significations données sous la formule (I) dans la revendication 1 et Met² a la signification donnée sous la formule (XVII) dans la revendication 10.

15. Composés de formule (XIX) 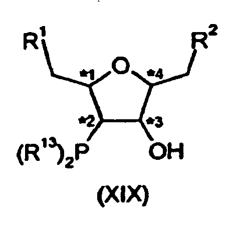 dans laquelle R¹, R² et R¹³ ont les significations données sous la formule (I) dans la revendication 1.

16. Composés de formule (XXa) 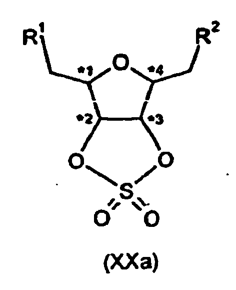 dans laquelle R¹ et R² ont les significations données sous la formule (I) dans la revendication 1.

17. Composés de formule (XXIa) 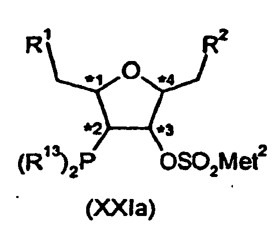 dans laquelle R¹, R² et R¹³ ont les significations données sous la formule (I) dans la revendication 1 et Met² a la signification donnée sous la formule (XVII) dans la revendication 10.

18. Composés de formule (XXIb) 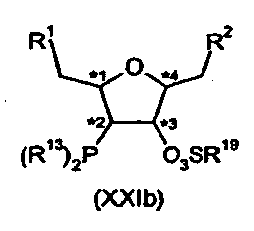 dans laquelle R¹, R² et R¹³ ont les significations données sous la formule (I) dans la revendication 1 et R¹⁹ représente un groupe alkyle en C₁-C₁₂, fluoroalkyle en C₁₋₁₂, arylalkyle en C₅-C₂₅ ou aryle en C₄-C₂₄.

19. Complexes de métaux de transition contenant des composés selon au moins l'une des revendications 1 à 10.

20. Complexes de métaux de transition selon la revendication 19, **caractérisés en ce que** le métal de transition est choisi dans le groupe constitué par le ruthénium, l'osmium, le cobalt, le rhodium, l'iridium, le nickel, le palladium, le platine et le cuivre.

21. Complexes de métaux de transition selon au moins l'une des revendications, 19 et 20, **caractérisés en ce que** le rapport molaire du métal de transition aux composés selon au moins l'une des revendications 1 à 10 est de 1:1.

22. Complexes de métaux de transition selon la revendication 20, **caractérisés en ce qu'**ils correspondent à la formule (XXIII)
[(I)L¹₂M] (XXIII)
dans laquelle (I) représente des composés de formule (I) ayant la signification donnée dans la revendication 1 et
M représente le rhodium ou l'iridium et
L¹ représente chaque fois un groupe alcène en C₂-C₁₂ ou un nitrile ou
L¹₂ représente ensemble un diène en C₄-C₁₂.

23. [Rh(cod)(2,3-bis-O-(diphénylphosphino)-1,6-di-O-(triphénylméthyl)-2,5-anhydro-D-mannitol)]BF₄, [Rh(cod)(2,3-bis-O-(diphénylphosphino)-1,6-di-O-(tert-butyldiphénylsilyl)-2,5-anhydro-D-mannitol)]-BF₄, [Rh(cod)(2,3-bis-O-(diphénylphosphino)-1,6-didésoxy-2,5-anhydro-D-mannitol)]BF₄ et [Ir(cod)-(2,3-bis-O-(diphénylphosphino)-1,6-di-O-(tert-butyldiphénylsilyl)-2,5-anhydro-D-mannitol)]BF₄.

24. Complexes de métaux de transition selon au moins l'une des revendications 19 à 21, **caractérisés en ce qu'**ils sont obtenus par réaction de composés contenant des métaux de transition et de composés selon une ou plusieurs des revendications 1 à 10.

25. Complexes de métaux de transition selon la revendication 24, **caractérisés en ce qu'**on utilise en tant que composés contenant des métaux de transition :
des composés contenant des métaux de transition de formule (XXIIa)
M(An¹)_{q} (XXIIa)
dans laquelle
M représente le rhodium, l'iridium, le ruthénium, le nickel, le palladium, le platine ou le cuivre et
An¹ représente l'ion chlorure, bromure, acétate, nitrate, méthanesulfonate, trifluorométhanesulfonate ou acétylacétonate et
q représente 3 pour le rhodium, l'iridium et le ruthénium, 2 pour le nickel, le palladium et le platine et 1 pour le cuivre,
ou des composés contenant des métaux de transition de formule (XXIIb)
M(An²)_{q}L¹₂ (XXIIb)
dans laquelle
M représente le ruthénium, l'iridium, le nickel, le palladium, le platine ou le cuivre et
An² représente l'ion chlorure, bromure, acétate, méthanesulfonate ou trifluorométhanesulfonate, tétrafluoroborate ou hexafluorophosphate, perchlorate, hexafluoroantimonate, tétra(bis-3,5-trifluorométhylphényl)borate ou tétraphénylborate et
q représente 1 pour le rhodium et l'iridium, 2 pour le ruthénium, le nickel, le palladium et le platine et 1 pour le cuivre,
L¹ représente chaque fois un groupe alcène en C₂-C₁₂ ou
L¹₂ représentent ensemble un diène en C₄₋₁₂,
ou des composés contenant des métaux de transition de formule (XXIIc)
[ML²An¹₂]₂ (XXIIc)
dans laquelle
M représente le ruthénium et
L² représente des radicaux aryle ou le groupe cyclo-octadiène, norbornadiène ou méthylallyle,
ou des composés contenant des métaux de transition de formule (XXIId),
Met³_{q}[M (An³)₄] (XXIId)
dans laquelle
M représente le palladium, le nickel, l'iridium ou le rhodium et
An³ représente l'ion chlorure ou bromure et
Met³ représente le lithium, le sodium, le potassium, l'ammonium ou un dérivé organique d'ammonium et
q représente 3 pour le rhodium et l'iridium, 2 pour le nickel, le palladium et le platine,
ou des composés contenant des métaux de transition de formule (XXIIe),
[M(L³)₂]An⁴ (XXIIe)
dans laquelle
M représente l'iridium ou le rhodium et
L³ représente un diène en C₄-C₁₂ et
An⁴ représente un anion non coordiné ou faiblement coordiné
ou
le Ni(1,5-cyclo-octadiène)₂, la Pd₂(dibenzydilène-acétone)₃, le Pd[PPh₃]₄, le cyclopentadiényl₂Ru, le Rh(acac)-(CO)₂, le Ir(pyridine)₂(1,5-cyclo-octadiène), le Cu(phényl)Br, le Cu(phényl)Cl, Cu(phényl)I, le Cu(PPh₃)₂Br, le [Cu(CH₃CN)₄]BF₄ et le [Cu(CH₃CN)₄]PF₆ ou des complexes polynucléaires pontés comme par exemple [Rh(1,5-cyclo-octadiène)Cl]₂, [Rh(1,5-cyclo-octadiène)Br]₂, [Rh(éthène)₂Cl]₂ ou [Rh(cyclo-octène)₂Cl]₂.

26. Complexes de métaux de transition selon la revendication 25, **caractérisés en ce qu'**on utilise en tant que composés contenant des métaux de transition : [Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bis-trifluorométhylphényle), [Rh(cod)₂]SbF₆, RuCl₂(cod), [(cymène)RuCl₂]₂, [(benzène)RuCl₂]₂, [(mésityl)RuCl₂]₂, [(cymène)RuBr₂]₂, [(cymène)RuI₂]₂, [(cymène)Ru(BF₄)₂]₂, [(cymène)Ru(PF₆)₂]₂, [(cymène)Ru(BAr₄)₂]₂ (Ar = 3,5-bis-trifluorométhylphényle), [(cymène)Ru(SbF₆)₂]₂, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bis-trifluorométhylphényle), RuCl₃, NiCl₃, RhCl₃, PdCl₂, PdBr₂, Pd(OAc)₂, Pd₂(dibenzylidène-acétone)₃, Pd(acétylacétonate)₂, CuOTf, CuI, CuCl, Cu(OTf)₂, CuBr, CuI, CuBr₂, CuCl₂, CuI₂, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bis-trifluorométhylphényle), [Rh(nbd)₂]SbF₆, RuCl₂(nbd), [Ir(nbd)₂]PF₆, [Ir(nbd)₂]ClO₄, [Ir(nbd)₂]SbF₆, [Ir(nbd)₂]BF₄, [Ir(nbd)₂]OTf, [Ir(nbd)₂]BAr₄ (Ar = 3,5-bis-trifluorométhylphényle), Ir(pyridin)₂(nbd), [Ru(DMSO)₄Cl₂], [Ru(CH₃CN)₄Cl₂], [Ru(PhCN)₄Cl₂], [Ru(cod)Cl₂]ₙ, [Ru(cod)₄(méthallyl)₂], [Ru(acétylacétonate)₃].

27. Complexes de métaux de transition selon la revendication 26, **caractérisés en ce qu'**on utilise en tant que composés contenant des métaux de transition :
[Rh(cod)Cl]₂, [Rh(cod)Br]₂, [Rh(cod)₂]ClO₄, [Rh(cod)₂]BF₄, [Rh(cod)₂]PF₄, [Rh(cod)₂]ClO₆, [Rh(cod)₂]OTf, [Rh(cod)₂]BAr₄ (Ar = 3,5-bis-trifluorométhylphényle), [Rh(cod)₂]SbF₆, [Rh(nbd)Cl]₂, [Rh(nbd)Br]₂, [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bis-trifluorométhylphényle), [Rh(nbd)₂]SbF₆, [Ir(cod)Cl]₂, [Ir(cod)₂]PF₆, [Ir(cod)₂]ClO₄, [Ir(cod)₂]SbF₆, [Ir(cod)₂]BF₄, [Ir(cod)₂]OTf, [Ir(cod)₂]BAr₄ (Ar = 3,5-bis-trifluorométhylphényle).

28. Complexes de métaux de transition selon au moins l'une des revendications 23 à 27, **caractérisés en ce que** la quantité des composés utilisés contenant des métaux de transition va de 25 à 200 % en moles, par rapport au composé selon une ou plusieurs des revendications 1 à 10 qui est utilisé.

29. Utilisation de complexes de métaux de transition selon une ou plusieurs des revendications 19 à 28, pour la préparation de composés enrichis en stéréoisomères.

30. Utilisation selon la revendication 29, **caractérisée en ce que** les composés enrichis en stéréoisomères sont obtenus par additions 1,4 asymétriques, hydroformylations asymétriques, hydrocyanurations asymétriques, réactions de Heck asymétriques et hydrogénations asymétriques.

31. Utilisation selon une ou plusieurs des revendications 29 et 30, **caractérisée en ce que** les composés enrichis en stéréoisomères sont utilisés pour la préparation de substances actives dans des médicaments et des produits phytosanitaires ou de produits intermédiaires de ces deux classes.

32. Utilisation de complexes de métaux de transition selon une ou plusieurs des revendications 19 à 28, en tant que catalyseurs.

33. Procédé pour la préparation de composés enrichis en stéréoisomères, par hydrogénations catalytiques d'oléfines, d'énamines, d'énamides, d'imines ou de cétones; des additions 1,4, des hydroformylations, des hydrocyanurations ou des réactions de Heck, **caractérisé en ce qu'**on utilise en tant que catalyseurs ceux qui contiennent des complexes de métaux de transition selon une ou plusieurs des revendications 19 à 28.

34. Procédé selon la revendication 33, **caractérisé en ce que** la quantité des complexes de métaux de transition utilisés va de 0,001 à 5 % en moles, par rapport au substrat utilisé.

35. Procédé selon une ou plusieurs des revendications 33 à 35, **caractérisé en ce que** les composés enrichis en stéréoisomères sont obtenus par hydrogénation catalytique d'oléfines, d'énamides ou d'imines.

36. Procédé selon une ou plusieurs des revendications 33 à 35, **caractérisé en ce qu'**on opère à une température de -20°C à 200°C.

37. Procédé selon une ou plusieurs des revendications 34 à 36, **caractérisé en ce que** la pression d'hydrogène va de 0,1 à 200 bars.

38. Catalyseurs contenant des complexes de métaux de transition selon une ou plusieurs des revendications 19 à 28.
